# EUROPEAN PATENT APPLICATION

(11) **EP 4 763 126 A1**
(43) Date of publication of application: **24.06.2026**
(21) Application number: 25223677.3
(22) Date of filing: 16.12.2025
(51) Int. Cl.: A61B 34/30, A61B 34/00, A61B 34/32

(54) **SYSTEMS AND METHODS EMPLOYING MACHINE LEARNING MODELS TO IMPROVE SURGICAL ROBOTIC PERFORMANCE**

(30) Priority: 20.12.2024 US 202463737587 P
(71) Applicant: MAKO Surgical Corp., Weston, FL 33331 (US)
(72) Inventor: Talasaz, Ali, Plantation, FL, 33325-2514 (US)
(74) Representative: Röthinger, Rainer

(57) **Abstract**

Robotic surgical systems and methods involving employing machine learning models to improve surgical robotic performance in task space and/or joint space. The robotic manipulator includes a plurality of links and joints and supports a surgical tool. A control system is coupled to the robotic manipulator. For task space control, the control system computes haptic forces that are intended to constrain specified degrees of freedom of the surgical tool and implements the machine learning model to refine the computed haptic forces in attempt to achieve constraint of the surgical tool according to the specified degrees of freedom. For joint space control, the control system computes joint torques for the joints of the robotic manipulator and implement the machine learning model to refine the computed joint torques in attempt to achieve desired joint positions. The machine learning models may use data-driven control schemes.

## Description

### CROSS-REFERENCE TO RELATED APPLICATIONS

The subject application claims priority to and all the benefits of U.S. Provisional Patent App. No. 63/737,587, filed December 20, 2024, the entire contents of which are hereby incorporated by reference.

### BACKGROUND

Surgical robotic systems typically include a manipulator that supports and moves a surgical tool to assist with performing a surgical procedure on a surgical site. Such manipulators are commonly controlled by the surgeon in a collaborative manner.

Accuracy of the surgical tool, particularly relative to the surgical site, is immensely important. Yet, many times, the joints of the manipulator and/or the surgical tool may not actually move in the commanded manner. Inaccuracies of only a few millimeters can cause sub-optimal control or performance of the manipulator and/or the surgical tool. As a result, such inaccuracies may cause disturbances to the surgeon, or worse, complications during surgery or a sub-optimal surgical outcome for the patient.

Certain errors, such as linear errors, are simpler for robotic control systems to address by using linear (e.g., PD or PID) controllers or calibration schemes. However, non-linear errors, such as those due to variant inertia, friction, joint flexibility, backlash, external disturbances, and tool interaction, are often the source of robotic or surgical tool inaccuracy. Non-linear errors are complex, difficult to predict and can result in non-diminishing steady state errors affecting tool accuracy. Despite this, conventional surgical robotic control systems usually settle for compensating only for linear errors, e.g., due to limited computational resources or additional system cost. However, simply compensating for linear errors may not be sufficient to provide the high level of tool accuracy needed for robotic surgery. Moreover, existing surgical robotic control systems typically rely on pre-defined control models to account for the robotic behavior. However, such pre-defined control models are inadequate to accurately identify or predict non-linear robotic behavior and cannot be updated or optimized to learn from environmental interactions.

Such errors can manifest in the task space and/or in the joint space of the robot. In the task space, the described errors can arise during robotic application of "active constraints" on the surgical tool, for example. Active constraints provide the surgeon with resistance or guidance of the surgical tool by actively restricting a surgeon's movement of the surgical tool in a specified manner. For example, active constraints can constrain certain degrees of freedom (DOF) of the surgical tool so that the surgical tool will feel stiff if the surgeon attempts to move the tool in one of the constrained DOF. However, the traditional control approaches may not provide desirably stiff active constraints during robotics-assisted procedures. For robotic arthroplasty, an insufficiently stiff active constraint may result in proud or deep cuts (inter-cut error) in the bone, leaving the surgeon with no choice but to perform undesirable corrections, such as cementing the implant. Moreover, prior control loops can exhibit aggressively high gains, and if the control loop fails to adequately resolve steady state error, the commanded constraints on the surgical tool may result in unpredictable tool oscillations resulting in a sensed "lack of control" of the tool for the surgeon and potential risk to the patient.

In the joint space, the joints of the manipulator are commanded to move to joint positions by application of commanded torques on the respective actuators of the joints. However, non-linearities may cause actual joint positions to differ from the commanded joint positions. As a result, the joints fail to move as desired, potentially resulting in inaccuracies of the surgical tool and inability to address errors throughout the entire workspace of the manipulator. Similarly, traditional control approaches for surgical robotics fail to provide sufficient compensation for non-linear joint errors.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical system is provided comprising: a robotic manipulator comprising a plurality of links and joints; a surgical tool coupled to the robotic manipulator; and a control system coupled to the robotic manipulator and being configured to: compute haptic forces that are intended to constrain specified degrees of freedom of the surgical tool; and implement a data-driven control model that is configured to refine the computed haptic forces in attempt to achieve constraint of the surgical tool according to the specified degrees of freedom.

According to a second aspect, a surgical system is provided comprising: a robotic manipulator comprising a plurality of links and joints; a surgical tool coupled to the robotic manipulator; and a control system coupled to the robotic manipulator and being configured to: compute haptic forces that are intended to constrain specified degrees of freedom of the surgical tool; and receive and process prior surgical tool poses and prior forces acting on the surgical tool to generate a control policy; and utilize the control policy to generate a refinement to the haptic forces in attempt to achieve constraint of the surgical tool according to the specified degrees of freedom.

According to a third aspect, a surgical system is provided comprising: a robotic manipulator comprising a plurality of links and joints; a surgical tool coupled to the robotic manipulator; a control system coupled to the robotic manipulator and being configured to: compute haptic forces that are intended to constrain specified degrees of freedom (DOF) of the surgical tool; based on the haptic forces, generate commanded joint torques to control the robotic manipulator to move to constraint poses in attempt to actively constrain the specified DOF of the surgical tool; measure actual joint torques generated responsive to movement of the robotic manipulator to the constraint poses; convert the actual joint torques into measured forces for the specified DOF; acquire prior measured forces; acquire prior constraint poses; obtain a desired constraint pose of the surgical tool according to the specified DOF; input the prior measured forces, the prior constraint poses, and the desired constraint pose into a machine learning model that is configured to optimize a cost function and generate an output comprising a refinement to the haptic forces in attempt to achieve the desired constraint pose of the surgical tool according to the specified DOF.

According to a fourth aspect, a surgical system is provided comprising: a robotic manipulator comprising a plurality of links and joints; a surgical tool coupled to the robotic manipulator; a control system coupled to the robotic manipulator and being configured to: compute haptic forces to constrain specified degrees of freedom (DOF) of the surgical tool; based on the haptic forces, generate commanded joint torques to control the robotic manipulator to move to constraint poses to actively constrain the specified DOF of the surgical tool; measure actual joint torques generated responsive to movement of the robotic manipulator to the constraint poses; convert the actual joint torques into measured forces for the specified DOF; acquire prior measured forces; acquire prior constraint poses; obtain a desired constraint pose of the surgical tool according to the specified DOF; input the prior measured forces, the prior constraint poses, and the desired constraint pose into a machine learning model that is configured to optimize a cost function and generate an output comprising a refinement to the haptic forces to maintain the desired constraint pose of the surgical tool according to the specified DOF.

According to a fifth aspect, a surgical system is provided comprising: a robotic manipulator comprising a plurality of links and joints; a surgical tool coupled to the robotic manipulator; a control system coupled to the robotic manipulator and being configured to: compute haptic forces to constrain specified degrees of freedom of the surgical tool; and implement a data-driven control model that is configured to refine the computed haptic forces to maintain constraint of the surgical tool according to the specified degrees of freedom.

According to a sixth aspect, a surgical system is provided comprising: a robotic manipulator comprising a plurality of links and joints; a surgical tool coupled to the robotic manipulator; a control system coupled to the robotic manipulator and being configured to: implement a data-driven control model that is configured to receive and process prior surgical tool poses and prior forces acting on the surgical tool to generate a control policy; and utilize the control policy to compute haptic forces that are intended to constrain specified degrees of freedom of the surgical tool.

According to a seventh aspect, a surgical system is provided comprising: a robotic manipulator comprising a plurality of links and joints; a surgical tool coupled to the robotic manipulator; a control system coupled to the robotic manipulator and being configured to: compute haptic forces to constrain the surgical tool relative to a virtual boundary; and implement a data-driven control model that is configured to refine the computed haptic forces to maintain constraint of the surgical tool according to the virtual boundary.

According to an eighth aspect, a surgical system is provided comprising: a robotic manipulator comprising a plurality of links and joints; a surgical tool coupled to the robotic manipulator; a control system coupled to the robotic manipulator and being configured to: compute haptic forces that are intended to constrain the surgical tool relative to a virtual boundary; based on the haptic forces, generate commanded joint torques to control the robotic manipulator to move to constraint poses in attempt to actively constrain the surgical tool relative to the virtual boundary; measure actual joint torques generated responsive to movement of the robotic manipulator to the constraint poses; convert the actual joint torques into measured forces for the specified DOF; acquire prior measured forces; acquire prior constraint poses; obtain a desired constraint pose of the surgical tool relative to the virtual boundary; input the prior measured forces, the prior constraint poses, and the desired constraint pose into a machine learning model that is configured to optimize a cost function and generate an output comprising a refinement to the haptic forces in attempt to achieve the desired constraint pose of the surgical tool relative to the virtual boundary.

According to a seventh aspect, a surgical system is provided comprising: a robotic manipulator comprising a plurality of links and joints; a surgical tool coupled to the robotic manipulator; and a control system coupled to the robotic manipulator and being configured to: compute joint torques for the joints of the robotic manipulator; and implement a data-driven control model that is configured to refine the computed joint torques in attempt to achieve desired joint positions.

According to a ninth aspect, a surgical system is provided comprising: a robotic manipulator comprising a plurality of links and joints; a surgical tool coupled to the robotic manipulator; and a control system coupled to the robotic manipulator and being configured to: compute joint torques for the joints of the robotic manipulator; receive and process prior joint positions and prior joint torques to generate a control policy; and utilize the control policy to generate a refinement to the computed joint torques in attempt to achieve desired joint positions.

According to a tenth aspect, a surgical system is provided comprising: a robotic manipulator comprising a plurality of links and joints; a control system coupled to the robotic manipulator and being configured to: compute joint torques for the joints of the robotic manipulator; control the joints of the robotic manipulator in accordance with the computed joint torques to move the joints to joint positions; acquire prior measured joint torques; acquire prior measured joint positions; obtain desired joint positions for the joints; input the prior measured joint torques, the prior measured joint positions, and the desired joint positions into a machine learning model that is configured to optimize a cost function and generate an output comprising a refinement to the computed joint torques in attempt to achieve the desired joint positions.

According to an eleventh aspect, a surgical system is provided comprising: a robotic manipulator comprising a plurality of links and joints; a control system coupled to the robotic manipulator and being configured to: compute joint torques for the joints of the robotic manipulator; control the joints of the robotic manipulator in accordance with the computed joint torques to move the joints to joint positions; acquire prior measured joint torques; acquire prior measured joint positions; obtain desired joint positions for the joints; input the prior measured joint torques, the prior measured joint positions, and the desired joint positions into a machine learning model that is configured to optimize a cost function and generate an output comprising a refinement to the computed joint torques to maintain the desired joint positions.

According to a twelfth aspect, a surgical system is provided comprising: a robotic manipulator comprising a plurality of links and joints; a surgical tool coupled to the robotic manipulator; and a control system coupled to the robotic manipulator and being configured to: compute joint torques for the joints of the robotic manipulator; and implement a data-driven control model that is configured to refine the computed joint torques to maintain desired joint positions.

According to a thirteenth aspect, a surgical system is provided comprising: a robotic manipulator comprising a plurality of links and joints; a surgical tool coupled to the robotic manipulator; a control system coupled to the robotic manipulator and being configured to: implement a data-driven control model that is configured to receive and process prior joint positions and prior joint torques to generate a control policy; and utilize the control policy to compute joint torques for the joints of the robotic manipulator.

A computer-implemented method is provided for performing any of the steps implemented by the surgical system or the control system of any one or more of the preceding aspects. A non-transitory computer readable medium or computer program product is provided, comprising instructions, which when executed by one or more processors, are configured to implement the surgical system or the control system of any one or more of the preceding aspects. Also provided is the control system of any one or more of the preceding aspects.

Any of the aspects described above can be combined in part, or in whole. Any of the above aspects can be utilized in part, or in whole, with any of the following implementations:

The robotic manipulator can be operated in a manual mode wherein the robotic manipulator is configured to move the surgical tool responsive to external forces/torques applied to the surgical tool by a user. The robotic manipulator can be operated in an automated mode wherein the robotic manipulator is configured to automatically move the surgical tool along a predetermined tool path. The robotic manipulator can be operated in a guided manual mode wherein the robotic manipulator is configured to move the surgical tool along a predetermined tool path responsive to external forces/torques applied to the surgical tool by a user.

The machine learning model can be a data-driven control model, a model-free adaptive control model, a model-free predictive control model, or any variation of reinforcement learning. The machine learning model can be configured to define or generate a refinement control policy. The machine learning model can include a neural network. The control system can utilize the machine learning model to optimize the cost function by being configured to minimize one or more of the following: future residual errors, oscillation in constraint poses, and energy injected into the robotic manipulator.

The refinement control policy can be generated based on prior poses/positions of components of robotic manipulator and prior forces/torques measured by components of the robotic manipulator, such as prior measured tool forces and prior tool constraint poses and/or prior measured joint torques and the prior measured joint positions. The refinement control policy can be generated based on a target value, such as a desired constraint pose or desired joint positions for the joints. The control system can input the prior poses/positions, prior forces/torques and a desired pose/position into the neural network. The machine learning model can generate the refinement by applying the prior measured forces, the prior constraint poses, and the desired constraint pose to the refinement control policy. The machine learning model can generate the refinement by applying the prior measured joint torques, the prior measured joint positions, and the desired joint positions to the refinement control policy.

The prior measured forces or measured joint torques can include forces/torques derived from: the haptic forces, computed joint torques, and/or external disturbance(s) acting on the surgical tool or robotic manipulator. The control system can utilize the refinement of the haptic forces to control the robotic manipulator to move to refined constraint poses in attempt to achieve the desired constraint pose of the surgical tool according to the specified DOF and/or in attempt to achieve the desired joint positions.

In any one or more of the manual mode, the automated mode, or the guided manual mode, the control system can utilize the refinements in attempt to actively constrain the specified DOF of the surgical tool to achieve or attempt to achieve the desired joint positions.

The haptic forces can comprise a haptic force component for each of the specified DOF. The refinement can include a force refinement to be added to the haptic force component for each of the specified DOF. The computed joint torques can comprise a torque value for each active joint of the robotic manipulator. The refinement can include a torque refinement to be added to the torque value for each active joint of the robotic manipulator. The control system can apply a range defining a minimum value and a maximum value for the refinement. The control system can apply the refinement only in response to the refinement falling within the range.

The control system can implement a haptic control model configured to compute the haptic forces that are intended to constrain the specified DOF of the surgical tool in a task space of the robotic manipulator. The haptic control model can be one or more of a spring-damper model; a proportional-derivative (PD) model; or an impulse model. The haptic control model can compute the haptic forces that are intended to constrain the specified DOF of the surgical tool relative to a haptic object. The haptic object can be a line, and the haptic control model can compute the haptic forces that are intended to constrain at least four specified DOF of the surgical tool relative to the haptic line. The at least four specified DOF comprise at least two rotational DOF and two translational DOF. The haptic object can be a haptic plane. The haptic control model can compute the haptic forces that are intended to constrain at least three specified DOF of the surgical tool relative to the haptic plane. The at least three specified DOF comprise two rotational DOF and at least one translational DOF. The haptic object can be a haptic volume. The haptic control model can compute the haptic forces that are intended to constrain up to three specified DOF of the surgical tool relative to the haptic volume. The up to three specified DOF comprise up to three rotational DOF.

The control system can implement a long short-term memory (LSTM). The LSTM can be coupled to an input of the machine learning model. The LSTM can be or include a recurrent neural network (RNN). The LSTM can receive values of the prior measured forces and values of the prior constraint poses and/or receive values of the prior measured joint torques and values of the prior measured joint positions. The LSTM can modify weights of the RNN to learn long-term dependencies among the values and to selectively retain or discard the values.

The neural network can generate an output that comprises an identification of a state of the robotic manipulator. The control system can input the target value, e.g., desired constraint pose(s) and/or the desired joint position(s), into the machine learning model. The machine learning model can implement an optimizer. The optimizer can be configured to input the identified state and the target value into the cost function and optimize the cost function to define the refinement control policy. The neural network can generate an output that comprises predicted states of the robotic manipulator, which optionally can be over a defined future horizon. The optimizer can be configured to input the predicted states and the target value into the cost function and optimize the cost function to define N future refinement control policies. The optimizer can select the refinement control policy based on the first of the N future refinement control policies. The control system can obtain a most-recent refinement to the haptic forces and/or computed joint torques. The control system can input the most-recent refinement into the neural network. The neural network can process the prior measured forces, the prior constraint poses, the desired constraint poses, and the most-recent refinement to define the refinement control policy. The neural network can process the prior measured joint torques, the prior measured joint positions, the desired joint positions, and the most-recent refinement to define the refinement control policy.

The control system can predict future outputs, such as future constraint poses and/or future joint positions. The future outputs can be input into the machine learning model. The control system can utilize the machine learning model to optimize the cost function based on the target value and the future outputs to determine one or more future refinements to the haptic forces and/or computed joint torques.

### DESCRIPTION OF THE DRAWINGS

Advantages of the present disclosure will be readily appreciated as the same becomes better understood by reference to the following detailed description when considered in connection with the accompanying drawings.
Figure 1 is a perspective view of a robotic surgical system, according to one implementation.
Figure 2 is a block diagram of an example control system for controlling the robotic surgical system.
Figure 3 is a functional block diagram of modules implemented by the control system, according to one implementation.
Figure 4 illustrates an example output of a boundary generator.
Figure 5 illustrates an example output of a path generator.
Figure 6 is a block diagram of the control system illustrating haptic force and joint torque computation process, according to one implementation.
Figures 7A, 7B, and 7C illustrate example active constraints and boundary constraints that can be applied to the surgical tool according to various haptic object geometries.
Figure 8 is a block diagram of the control system illustrating a machine learning model to control or refine haptic forces and/or joint torques, according to one implementation.
Figure 9 is a block diagram of the control system illustrating the machine learning model to refine haptic forces, according to one implementation.
Figure 10 is a block diagram of the machine learning model to refine haptic forces, according to one implementation.
Figure 11 is a block diagram of the machine learning model to refine haptic forces implemented using a form of model-free adaptive control, according to one implementation.
Figure 12 is a block diagram of the machine learning model to refine haptic forces implemented using a form of model-free predictive control, according to one implementation.
Figure 13 is a block diagram of the machine learning model to refine haptic forces implemented using a form of deep reinforcement learning, according to one implementation.
Figure 14 is a block diagram of the control system illustrating the machine learning model to refine joint torques, according to one implementation.
Figure 15 is a block diagram of the machine learning model to refine joint torques, according to one implementation.
Figure 16 is a block diagram of the machine learning model to refine joint torques implemented using a form of model-free adaptive control, according to one implementation.
Figure 17 is a block diagram of the machine learning model to refine joint torques implemented using a form of model-free predictive control, according to one implementation.
Figure 18 is a block diagram of the machine learning model to refine joint torques implemented using a form of deep reinforcement learning, according to one implementation.
Figure 19 is a table of example expressions and equations that the control system can utilize in haptic force and/or joint torque refinement calculations for any of the implementations described herein.

### DETAILED DESCRIPTION

### I. Example System Overview

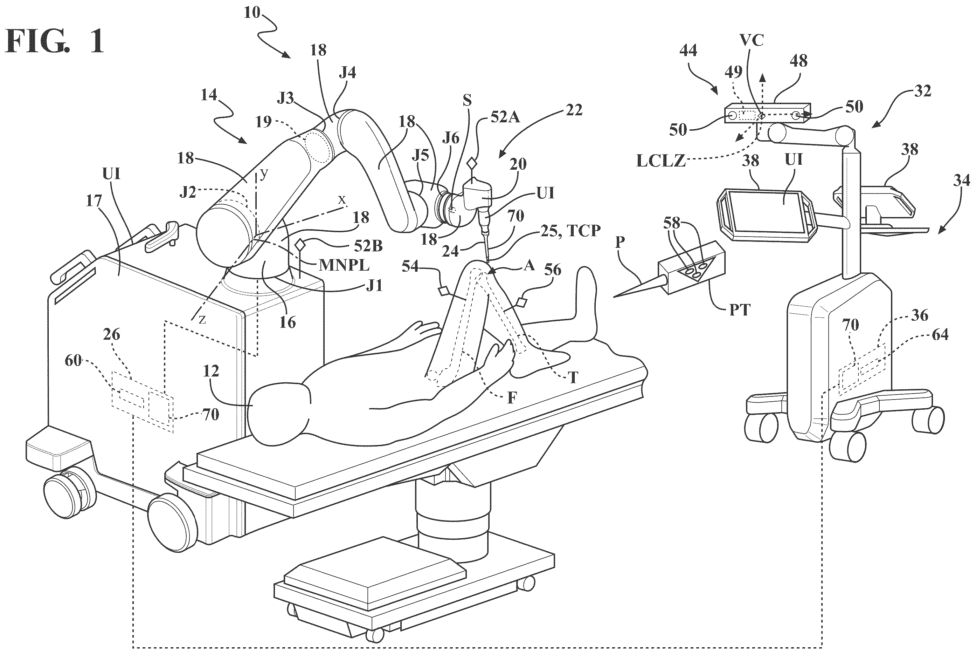

Referring to Figure 1, a robotic surgical system 10 is illustrated. The system 10 is useful for treating a surgical site or anatomical volume (A) of a patient 12, such as treating bone or soft tissue. In Figure 1, the patient 12 is undergoing a surgical procedure. The anatomy in Figure 1 includes a femur F and a tibia T of the patient 12. The surgical procedure may involve tissue removal or other forms of treatment. Treatment may include cutting, coagulating, lesioning the tissue, other in-situ tissue treatments, or the like. In some examples, the surgical procedure involves partial or total knee or hip replacement surgery, shoulder replacement surgery, spine surgery, or ankle surgery. In some examples, the system 10 is designed to cut away material to be replaced by surgical implants, such as hip and knee implants, including unicompartmental, bicompartmental, multicompartmental, or total knee implants. Some of these types of implants are shown in U.S. Patent Application Publication No. 2012/0330429, entitled, "Prosthetic Implant and Method of Implantation," the disclosure of which is hereby incorporated by reference. The system 10 and techniques disclosed herein may be utilized to perform other procedures, surgical or non-surgical, or may be utilized in industrial applications or other applications where robotic systems are utilized.

The system 10 includes a manipulator 14 or robotic manipulator. The manipulator 14 has a base 16 and plurality of links 18. A manipulator cart 17 supports the manipulator 14 such that the manipulator 14 is fixed to the manipulator cart 17. The links 18 collectively form one or more arms of the manipulator 14. The manipulator 14 may have a serial arm configuration (as shown in Figure 1), a parallel arm configuration, or any other suitable manipulator configuration. In other examples, more than one manipulator 14 may be utilized in a multiple arm configuration.

The manipulator 14 can include a passive (manually articulated) joint, such as planarly extending joint. For instance, the passive joint may by the distal most joint attached to the robotic arm comprising a plurality of active joints. The planarly extending joint can support a tool such as a saw blade to allow the user to manually move the saw blade along a plane. In this case, the tool is mechanically constrained to the plane by the mechanical constraints of the passive joint, while the pose of the plane is actively constrained by the active joints of the robotic arm. In other instances, one or more joints of the manipulator 14 could be controlled to constrain the surgical tool in certain degrees of freedom, while allowing the surgical tool free passive motion in other degrees of freedom or manners, such as a rotation about a point or a selected linear motion.

The manipulator 14 can exhibit kinematic redundancy wherein the tool positions are defined by up to 6DOF, but the robotic arm may operate in more than 6DOF. With such redundancy, the manipulator 14 can utilize null-space controls whereby the joints of the manipulator 14 can change positions while maintaining the pose of the tool 20. Null-space controls can also be used to avoid singularities.

In the example shown in Figure 1, the manipulator 14 comprises a plurality of joints J and a plurality of joint encoders 19 located at the joints J for determining position data of the joints J. For simplicity, only one joint encoder 19 is illustrated in Figure 1, although other joint encoders 19 may be similarly illustrated. The manipulator 14 according to one example has six joints J1-J6 implementing at least six-degrees of freedom (DOF) for the manipulator 14. However, the manipulator 14 may have any number of degrees of freedom and may have any suitable number of joints J and may have redundant joints.

The manipulator 14 need not require joint encoders 19 but may alternatively, or additionally, utilize motor encoders present on motors at each joint J. Also, the manipulator 14 need not require rotary joints, but may alternatively, or additionally, utilize one or more prismatic joints. Any suitable combination of joint types is contemplated.

The base 16 of the manipulator 14 is a portion of the manipulator 14 that provides a fixed reference coordinate system for other components of the manipulator 14 or the system 10 in general. The origin of a manipulator coordinate system MNPL is defined at the fixed reference of the base 16. The base 16 may be defined with respect to any suitable portion of the manipulator 14, such as one or more of the links 18. Alternatively, or additionally, the base 16 may be defined with respect to the manipulator cart 17, such as where the manipulator 14 is physically attached to the manipulator cart 17. In one example, the base 16 is defined at an intersection of the axes of joints J1 and J2. Thus, although joints J1 and J2 are moving components in reality, the intersection of the axes of joints J1 and J2 is nevertheless a virtual fixed reference pose, which provides both a fixed position and orientation reference and which does not move relative to the manipulator 14 and/or manipulator cart 17.

In other examples, the manipulator 14 can be a hand-held manipulator where the base 16 is a base portion of a tool (e.g., a portion held free-hand by the user) and the tool tip is movable relative to the base portion. The base portion has a reference coordinate system that is tracked, and the tool tip has a tool tip coordinate system that is computed relative to the reference coordinate system (e.g., via motor and/or joint encoders and forward kinematic calculations). Movement of the tool tip can be controlled to follow the path since its pose relative to the path can be determined. The hand-held manipulator 14 can be like that described and shown in US20230255701, entitled "Systems And Methods For Guiding Movement Of A Handheld Medical Robotic Instrument", the entire disclosure of which is hereby incorporated by reference.

The manipulator 14 and/or manipulator cart 17 house a manipulator controller 26, or other type of control unit. The manipulator controller 26 may comprise one or more computers, or any other suitable form of controller that directs the motion of the manipulator 14. The manipulator controller 26 may have a central processing unit (CPU), graphics processing unit (GPU) and/or other processors, memory, and storage. The manipulator controller 26 is loaded with software as described below. The processors could include one or more processors to control operation of the manipulator 14. The processors can be any type of microprocessor, multi-processor, and/or multi-core processing system. The manipulator controller 26 may additionally, or alternatively, comprise one or more microcontrollers, field programmable gate arrays, systems on a chip, discrete circuitry, and/or other suitable hardware, software, or firmware that is capable of carrying out the functions described herein. The term processor is not intended to limit any embodiment to a single processor. The manipulator 14 may also comprise a user interface UI with one or more displays and/or input devices (e.g., push buttons, keyboard, mouse, microphone (voice-activation), gesture control devices, touchscreens, etc.).

A tool 20 couples to the manipulator 14 and is movable relative to the base 16 to interact with the anatomy in certain modes. The tool 20 is a physical and surgical tool and is, or forms part of, an end effector 22 supported by the manipulator 14 in certain implementations. The tool 20 may be grasped by the user. One possible arrangement of the manipulator 14 and the tool 20 is described in U.S. Patent No. 9,119,655, entitled, "Surgical Manipulator Capable of Controlling a Surgical Instrument in Multiple Modes," the disclosure of which is hereby incorporated by reference. The manipulator 14 and the tool 20 may be arranged in alternative configurations. The tool 20 can be like that shown in U.S. Patent Application Publication No. 2014/0276949, filed on March 15, 2014, entitled, "End Effector of a Surgical Robotic Manipulator," hereby incorporated by reference.

The tool 20 can include an energy applicator 24 designed to contact and remove the tissue of the patient 12 at the surgical site. In one example, the energy applicator 24 is a bur 25. The bur 25 may be substantially spherical and comprise a spherical center, radius (r) and diameter. Alternatively, the energy applicator 24 may be a drill bit, a saw blade, an ultrasonic vibrating tip, or the like. The tool 20 and/or energy applicator 24 may comprise any geometric feature, e.g., perimeter, circumference, radius, diameter, width, length, volume, area, surface/plane, range of motion envelope (along any one or more axes), etc. The geometric feature may be considered to determine how to locate the tool 20 relative to the tissue at the surgical site to perform the desired treatment. In some of the embodiments described herein, a spherical bur having a tool center point (TCP) will be described for convenience and ease of illustration but is not intended to limit the tool 20 to any particular form. In other examples, the tool 20 does not include an energy applicator 24. For example, the tool 20 can be a slotted cut guide for a saw, a guide tube for receiving another tool, or the like.

The tool 20 may comprise a tool controller to control operation of the tool 20, such as to control power to the tool (e.g., to a rotary motor of the tool 20), control movement of the tool 20, control irrigation/aspiration of the tool 20, and/or the like. The tool controller may be in communication with the manipulator controller 26 or other components. The tool 20 may also comprise a user interface UI with one or more displays and/or input devices (e.g., push buttons, keyboard, mouse, microphone (voice-activation), gesture control devices, touchscreens, etc.). For example, one of the user input devices on the user interface UI of the tool 20 may be a tool input (e.g., switch or other form of user input device) that has first and second input states (see FIG. 1). The tool input can be actuated (e.g., pressed and held) by the user to be placed in the first input state and can be released to be placed in the second input state. The tool 20 may have a grip on which the tool input is located. In some versions, the tool input is a presence detector that detects the presence of a hand of the user, such as a momentary contact switch that switches between on/off states, a capacitive sensor, an optical sensor, or the like. The tool input is thus configured such that the first input state indicates that a user is actively engaging the tool 20 and the second input state indicates that the user has released the tool 20. The tool input may be a continuous activation device, i.e., inputs that must be continually actuated to allow motion of the tool 20 in the manual mode or the semi-autonomous mode, depending on which user input is actuated. For example, while the user is continually actuating the tool input, and the manual mode is enabled, the manipulator 14 will move in response to the input forces and torques applied by the user and the control system 60 will enforce the virtual boundary 71 to protect the patient anatomy. When the tool input is released, input from the force/torque sensor S may be disabled such that the manipulator 14 no longer responds to the forces and torques applied by the user to the tool 20.

The manipulator controller 26 controls a state (position and/or orientation) of the tool 20 (e.g., the TCP) with respect to a coordinate system, such as the manipulator coordinate system MNPL. The manipulator controller 26 can control (linear or angular) velocity, acceleration, or other derivatives of motion of the tool 20. The tool center point (TCP), in one example, is a predetermined reference point defined at the energy applicator 24. The TCP has a known, or able to be calculated (i.e., not necessarily static), pose relative to other coordinate systems. The geometry of the energy applicator 24 is known in or defined relative to a TCP coordinate system. The TCP may be located at the spherical center of the bur 25 of the tool 20 such that only one point is tracked. The TCP may be defined in various ways depending on the configuration of the energy applicator 24. The manipulator 14 could employ the joint/motor encoders, or any other non-encoder position sensing method, to enable a pose of the TCP to be determined. The manipulator 14 may use joint measurements to determine TCP pose and/or could employ techniques to measure TCP pose directly. The control of the tool 20 is not limited to a center point. For example, any suitable primitives, meshes, etc., can be utilized to represent the tool 20.

The system 10 further includes a navigation system 32. One example of the navigation system 32 is described in U.S. Patent No. 9,008,757, filed on September 24, 2013, entitled, "Navigation System Including Optical and Non-Optical Sensors," hereby incorporated by reference. The navigation system 32 tracks movement of various objects. Such objects include, for example, the manipulator 14, the tool 20 and the anatomy, e.g., femur F and tibia T. The navigation system 32 tracks these objects to gather state information of each object with respect to a (navigation) localizer coordinate system LCLZ. Coordinates in the localizer coordinate system LCLZ may be transformed to the manipulator coordinate system MNPL, and/or vice-versa, using transformations.

The navigation system 32 includes a cart assembly 34 that houses a navigation controller 36, and/or other types of control units. A navigation user interface UI is in operative communication with the navigation controller 36. The navigation user interface includes one or more displays 38. The navigation system 32 is capable of displaying a graphical representation of the relative states of the tracked objects to the user using the one or more displays 38. The navigation user interface UI further comprises one or more input devices to input information into the navigation controller 36 or otherwise to select/control certain aspects of the navigation controller 36. Such input devices include interactive touchscreen displays. However, the input devices may include any one or more of push buttons, a keyboard, a mouse, a microphone (voice-activation), gesture control devices, and the like.

The navigation system 32 also includes a navigation localizer 44 coupled to the navigation controller 36. In one example, the localizer 44 is an optical localizer and includes a camera unit 46. The camera unit 46 has an outer casing 48 that houses one or more optical sensors 50. The localizer 44 may comprise its own localizer controller 49 and may further comprise a video camera VC.

The navigation system 32 includes one or more trackers. In one example, the trackers include a pointer tracker PT, one or more manipulator trackers 52A, 52B, a first patient tracker 54, and a second patient tracker 56. In the illustrated example of Figure 1, the manipulator tracker is rigidly attached to the tool 20 (i.e., tracker 52A), the first patient tracker 54 is firmly affixed to the femur F of the patient 12, and the second patient tracker 56 is firmly affixed to the tibia T of the patient 12. In this example, the patient trackers 54, 56 are firmly affixed to sections of bone. The pointer tracker PT is firmly affixed to a pointer P utilized for registering the anatomy to the localizer coordinate system LCLZ. The manipulator tracker 52A, 52B may be affixed to any suitable component of the manipulator 14, in addition to, or other than the tool 20, such as the base 16 (i.e., tracker 52B), or any one or more links 18 of the manipulator 14. The trackers 52A, 52B, 54, 56, PT may be fixed to their respective components in any suitable manner. For example, the trackers may be rigidly fixed, flexibly connected (optical fiber), or not physically connected at all (ultrasound), as long as there is a suitable (supplemental) way to determine the relationship (measurement) of that respective tracker to the object that it is associated with.

Any one or more of the trackers may include active markers 58. The active markers 58 may include light emitting diodes (LEDs). Alternatively, the trackers 52A, 52B, 54, 56, PT may have passive markers, such as reflectors, which reflect light emitted from the camera unit 46. Other suitable markers not specifically described herein may be utilized.

The localizer 44 tracks the trackers 52A, 52B, 54, 56, PT to determine a state of each of the trackers 52A, 52B, 54, 56, PT, which correspond respectively to the state of the object respectively attached thereto. The localizer 44 may perform known triangulation techniques to determine the states of the trackers 52, 54, 56, PT, and associated objects. The localizer 44 provides the state of the trackers 52A, 52B, 54, 56, PT to the navigation controller 36. In one example, the navigation controller 36 determines and communicates the state the trackers 52A, 52B, 54, 56, PT to the manipulator controller 26. As used herein, the state of an object includes, but is not limited to, data that defines the position and/or orientation of the tracked object or equivalents/derivatives of the position and/or orientation. For example, the state may be a pose of the object, and may include linear velocity data, and/or angular velocity data, and the like.

The navigation controller 36 may comprise one or more computers, or any other suitable form of controller. Navigation controller 36 may have a central processing unit (CPU), graphics processing unit (GPU) and/or other processors, memory, and storage. The processors can be any type of processor, microprocessor, or multi-processor system. The navigation controller 36 is loaded with software. The software, for example, converts the signals received from the localizer 44 into data representative of the position and orientation of the objects being tracked. The navigation controller 36 may additionally, or alternatively, comprise one or more microcontrollers, field programmable gate arrays, systems on a chip, discrete circuitry, and/or other suitable hardware, software, or firmware that is capable of conducting the functions described herein. The term processor is not intended to limit any embodiment to a single processor.

Although one example of the navigation system 32 is shown that employs triangulation techniques to determine object states, the navigation system 32 may have any other suitable configuration for tracking the manipulator 14, tool 20, and/or the patient 12.

In another example, the navigation system 32 and/or localizer 44 are ultrasound-based. For example, the navigation system 32 may comprise an ultrasound imaging device coupled to the navigation controller 36. The ultrasound imaging device images any of the aforementioned objects, e.g., the manipulator 14, the tool 20, and/or the patient 12, and generates state signals to the navigation controller 36 based on the ultrasound images. The ultrasound images may be 2-D, 3-D, or a combination of both. The navigation controller 36 may process the images in near real-time to determine states of the objects. The ultrasound imaging device may have any suitable configuration and may be different than the camera unit 46 as shown in Figure 1.

In another example, the navigation system 32 and/or localizer 44 are radio frequency (RF)-based. For example, the navigation system 32 may comprise an RF transceiver coupled to the navigation controller 36. The manipulator 14, the tool 20, and/or the patient 12 may comprise RF emitters or transponders attached thereto. The RF emitters or transponders may be passive or actively energized. The RF transceiver transmits an RF tracking signal and generates state signals to the navigation controller 36 based on RF signals received from the RF emitters. The navigation controller 36 may analyze the received RF signals to associate relative states thereto. The RF signals may be of any suitable frequency. The RF transceiver may be positioned at any suitable location to track the objects using RF signals effectively. Furthermore, the RF emitters or transponders may have any suitable structural configuration that may be much different than the trackers 52A, 52B, 54, 56, PT shown in Figure 1.

In yet another example, the navigation system 32 and/or localizer 44 are electromagnetically based. For example, the navigation system 32 may comprise an EM transceiver coupled to the navigation controller 36. The manipulator 14, the tool 20, and/or the patient 12 may comprise EM components attached thereto, such as any suitable magnetic tracker, electro-magnetic tracker, inductive tracker, or the like. The trackers may be passive or actively energized. The EM transceiver generates an EM field and generates state signals to the navigation controller 36 based upon EM signals received from the trackers. The navigation controller 36 may analyze the received EM signals to associate relative states thereto. Again, such navigation system 32 examples may have structural configurations that are different than the navigation system 32 configuration shown in Figure 1.

The navigation system 32 may have any other suitable components or structure not specifically recited herein. Furthermore, any of the techniques, methods, and/or components described above with respect to the navigation system 32 shown may be implemented or provided for any of the other examples of the navigation system 32 described herein. For example, the navigation system 32 may utilize solely inertial tracking or any combination of tracking techniques, and may additionally or alternatively comprise, fiber optic-based tracking, machine-vision tracking, and the like.

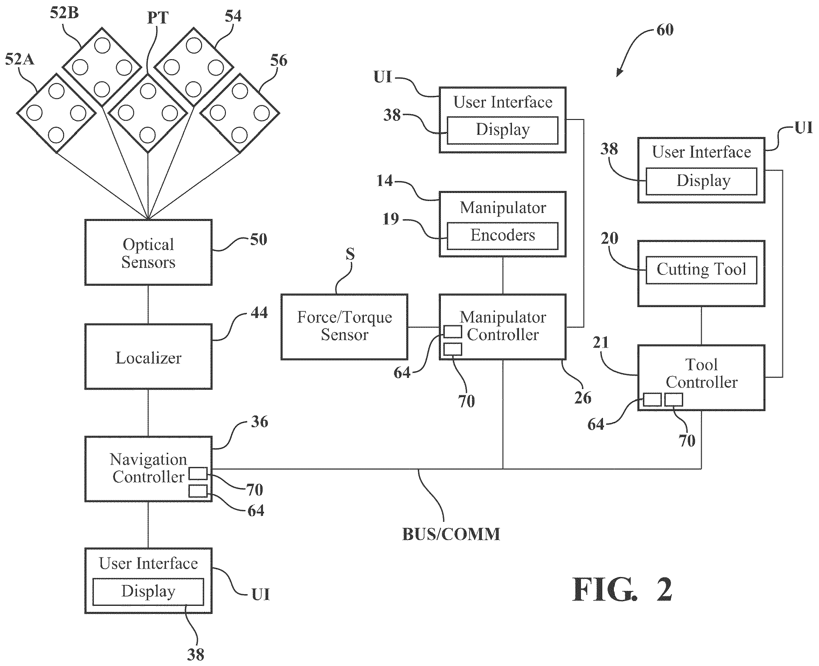

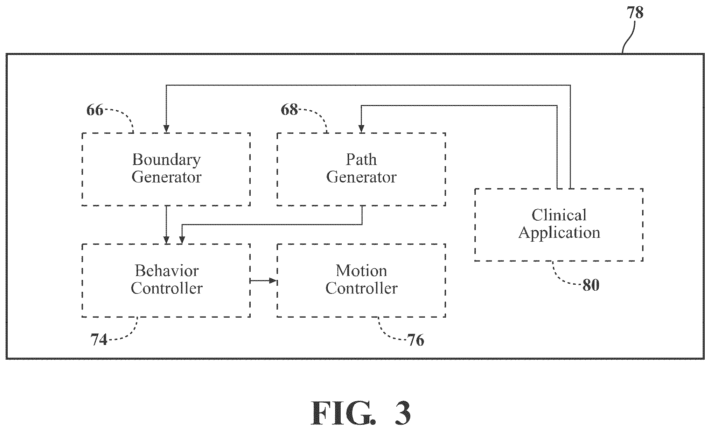

Referring to Figure 2, the system 10 includes a control system 60 that comprises, among other components, the manipulator controller 26, the navigation controller 36, and the tool controller 21. The control system 60 further includes one or more software programs and software modules shown in Figure 3. The software modules may be part of the program or programs that operate on the manipulator controller 26, navigation controller 36, tool controller 21, or any combination thereof, to process data to assist with control of the system 10. The software programs and/or modules include computer readable instructions stored in non-transitory memory 64 on the manipulator controller 26, navigation controller 36, tool controller 21, or a combination thereof, to be executed by one or more processors 70 of the controllers 21, 26, 36. The memory 64 may be any suitable configuration of memory, such as RAM, non-volatile memory, etc., and may be implemented locally or from a remote database. Additionally, software modules for prompting and/or communicating with the user may form part of the program or programs and may include instructions stored in memory 64 on the manipulator controller 26, navigation controller 36, tool controller 21, or any combination thereof. The user may interact with any of the input devices of the navigation user interface UI or other user interface UI to communicate with the software modules. The user interface software may run on a separate device from the manipulator controller 26, navigation controller 36, and/or tool controller 21.

The control system 60 may comprise any suitable configuration of input, output, and processing devices suitable for conducting the functions and methods described herein. The control system 60 may comprise the manipulator controller 26, the navigation controller 36, or the tool controller 21, or any combination thereof, or may comprise only one of these controllers. These controllers may communicate via a wired bus or communication network as shown in Figure 2, via wireless communication, or otherwise. The control system 60 may also be referred to as a controller. The control system 60 may comprise one or more microcontrollers, field programmable gate arrays, systems on a chip, discrete circuitry, sensors, displays, user interfaces, indicators, and/or other suitable hardware, software, or firmware that is capable of performing the functions described herein.

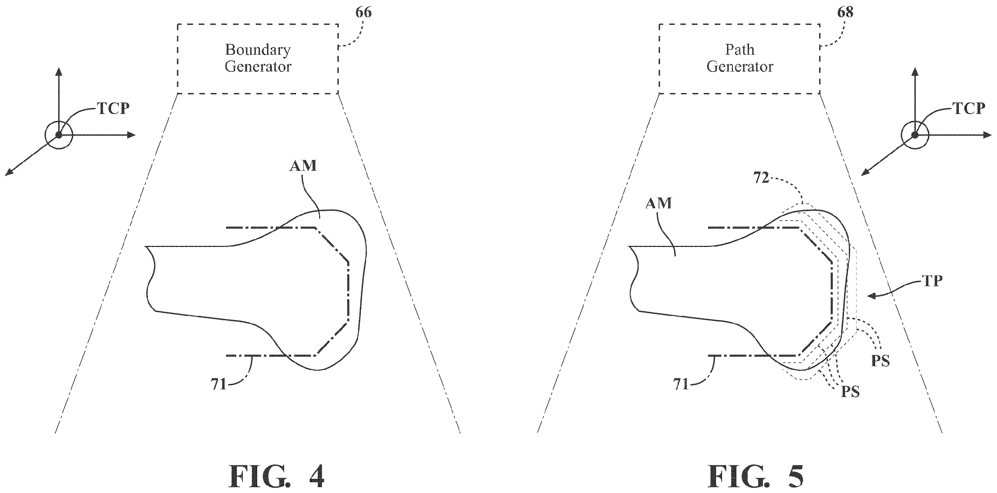

Referring to Figure 3, the software employed by the control system 60 includes a boundary generator 66. As shown in Figure 4, the boundary generator 66 is a software program or module that generates a virtual boundary 71 for constraining movement and/or operation of the tool 20. The virtual boundary 71 may be one-dimensional, two-dimensional, three-dimensional, and may comprise a point, line, axis, trajectory, plane, or other shapes, including complex geometric shapes. In some embodiments, the virtual boundary 71 is a surface defined by a triangle mesh. Such virtual boundaries 71 may also be referred to as virtual objects. The virtual boundaries 71 may be defined with respect to an anatomical model AM, such as a 3-D bone model. In the example of Figure 4, the virtual boundaries 71 are planar boundaries to delineate five planes for a total knee implant, and are associated with a 3-D model of the head of the femur F. The anatomical model AM is registered to the one or more patient trackers 54, 56 such that the virtual boundaries 71 become associated with the anatomical model AM. The virtual boundaries 71 may be implant-specific, e.g., defined based on a size, shape, volume, etc. of an implant and/or patient-specific, e.g., defined based on the patient's anatomy. The virtual boundaries 71 may be boundaries that are created pre-operatively, intra-operatively, or combinations thereof. In other words, the virtual boundaries 71 may be defined before the surgical procedure begins, during the surgical procedure (including during tissue removal), or combinations thereof. In any case, the control system 60 obtains the virtual boundaries 71 by storing/retrieving the virtual boundaries 71 in/from memory, obtaining the virtual boundaries 71 from memory, creating the virtual boundaries 71 pre-operatively, creating the virtual boundaries 71 intra-operatively, or the like.

The manipulator controller 26 and/or the navigation controller 36 track the state of the tool 20 relative to the virtual boundaries 71. In one example, the state of the TCP is measured relative to the virtual boundaries 71 for purposes of determining haptic forces to be applied to a virtual rigid body model via a virtual simulation 88 so that the tool 20 remains in a desired positional relationship to the virtual boundaries 71 (e.g., not moved beyond them). The results of the virtual simulation 88 are commanded to the manipulator 14. The control system 60 controls/positions the manipulator 14 in a manner that emulates the way a physical handpiece would respond in the presence of physical boundaries/barriers. The boundary generator 66 may be implemented on the manipulator controller 26. Alternatively, the boundary generator 66 may be implemented on other components, such as the navigation controller 36.

Referring to Figures 3 and 5, a path generator 68 is another software program or module run by the control system 60. In one example, the path generator 68 is run by the manipulator controller 26. The path generator 68 generates a tool path TP for the tool 20 to traverse. The tool path TP may comprise a plurality of path segments PS, or may comprise a single path segment PS. The path segments PS may be straight segments, curved segments, combinations thereof, or the like. The tool path TP may be defined with respect to the manipulator 14 coordinate system MNPL, localizer coordinate system LCLZ, coordinate system of the tool 20, coordinate system of the anatomy, or any combination thereof. The tool path TP can be virtually attached to the coordinate system of the respective object such that if the object were to move, the tool path TP will correspondingly move. The tool path TP may be implant-specific, e.g., defined based on a size, shape, volume, etc. of an implant and/or patient-specific, e.g., defined based on the patient's anatomy. The tool path TP can be associated with a virtual model of the anatomy and the virtual model and tool path can be registered to the anatomy using the navigation system 32. The control system 60 can generate or obtain the tool path TP by storing/retrieving the tool path TP in/from memory, creating the tool path TP pre-operatively, creating the tool path TP intra-operatively, or the like. The tool path TP may have any 3D shape, or combinations of shapes, such as circular, helical/corkscrew, linear, curvilinear, combinations thereof, and the like.

In one implementation, the tool path TP is defined as a guidance or alignment path. In one example, the tool path TP is for guiding the tool 20 to move to a location that positions the tool 20 for a start of the surgical procedure, or step. For instance, if the tool 20 is a saw blade, the tool path TP may be configured to guide the saw blade to align to a cut plane associated with the anatomy. If the tool 20 is a cutting bur, the tool path TP may be configured to guide the cutting bur to a starting point in preparation for automated cutting. A lead-in path could be virtually connected from the starting point to another cutting path for removal of tissue. The tool path TP may also enable the tool 20 to move along a predefined path of motion for purposes of registering components of the manipulator 14 to the navigation system 32. The tool path TP can be registered to the anatomy using the navigation system 32 such that the tool path TP is virtually fixed to the anatomy. This way, the tool path TP location in space will automatically be updated to account for any movement of the anatomy.

In another implementation, as shown in FIG. 5, the tool path TP is defined as a tissue removal path. One example of the tissue removal path described herein comprises a milling path 72. The term "milling path" generally refers to the path of the tool 20 in the vicinity of the target site for milling the anatomy and is not intended to require that the tool 20 be operably milling the anatomy throughout the entire duration of the path. For instance, as will be understood in further detail below, the milling path 72 may comprise sections or segments where the tool 20 transitions from one location to another without milling. Additionally, other forms of tissue removal along the milling path 72 may be employed, such as tissue ablation, and the like. The milling path 72 may be a predefined path that is created pre-operatively, intra-operatively, or combinations thereof. In other words, the milling path 72 may be defined before the surgical procedure begins, during the surgical procedure (including during tissue removal), or combinations thereof.

One example of a system and method for generating the virtual boundaries 71 and/or the milling path 72 is described in U.S. Patent No. 9,119,655, entitled, "Surgical Manipulator Capable of Controlling a Surgical Instrument in Multiple Modes," the disclosure of which is hereby incorporated by reference. In some examples, the virtual boundaries 71 and/or tool paths TP may be generated offline rather than on the manipulator controller 26 or navigation controller 36. Thereafter, the virtual boundaries 71 and/or tool paths TP may be utilized at runtime by the manipulator controller 26.

Referring to Figure 3, two additional software programs or modules run on the manipulator controller 26 and/or the navigation controller 36. One software module is a behavior controller 74. Behavior controller 74 can compute data that indicates the next commanded pose and/or orientation (e.g., pose) for the tool 20. In some cases, only the position of the TCP is output from the behavior controller 74, while in other cases, the position and orientation of the tool 20 is output. Output from the boundary generator 66, the path generator 68, and a force/torque sensor S may feed as inputs into the behavior controller 74 to determine the next commanded pose and/or orientation for the tool 20. The behavior controller 74 may process these inputs, along with one or more virtual constraints described further below, to determine the commanded pose. The behavior controller 74 can be implemented in an admittance control mode, wherein the robotic surgical system proactively generates the commanded position based on an output of a virtual rigid body simulation.

The second software module can include a motion controller 76. One aspect of motion control is the control of the manipulator 14. The motion controller 76 receives data defining the next commanded pose from the behavior controller 74. Based on these data, the motion controller 76 determines the next position of the joint angles of the joints J of the manipulator 14 (e.g., via inverse kinematics and Jacobian calculators) so that the manipulator 14 is able to position the tool 20 as commanded by the behavior controller 74, e.g., at the commanded pose. In other words, the motion controller 76 processes the commanded pose, which may be defined in Cartesian space, into joint angles of the manipulator 14, so that the manipulator controller 26 can command the joint motors accordingly, to move the joints J of the manipulator 14 to commanded joint angles corresponding to the commanded pose of the tool 20. In one version, the motion controller 76 regulates the joint angle of each joint J and continually adjusts the torque that each joint motor outputs to, as closely as possible, ensure that the joint motor drives the associated joint J to the commanded joint angle.

The boundary generator 66, path generator 68, behavior controller 74, and motion controller 76 may be sub-sets of a software program 78. Alternatively, each may be software programs that operate separately and/or independently in any combination thereof. The term "software program" is used herein to describe the computer-executable instructions that are configured to perform the various capabilities of the technical solutions described. For simplicity, the term "software program" is intended to encompass, at least, any one or more of the boundary generator 66, path generator 68, behavior controller 74, and/or motion controller 76. The software program 78 can be implemented on the manipulator controller 26, navigation controller 36, or any combination thereof, or may be implemented in any suitable manner by the control system 60.

A clinical application 80 may be provided to manage user interaction. The clinical application 80 handles many aspects of user interaction and coordinates the surgical workflow, including pre-operative planning, implant placement, registration, bone preparation visualization, and post-operative evaluation of implant fit, etc. The clinical application 80 is configured to output to the displays 38. The clinical application 80 may run on its own separate processor or may run alongside the navigation controller 36. In one example, the clinical application 80 interfaces with the boundary generator 66 and/or path generator 68 after implant placement is set by the user, and then sends the virtual boundary 71 and/or tool path TP returned by the boundary generator 66 and/or path generator 68 to the manipulator controller 26 for execution. Manipulator controller 26 executes the tool path TP as described herein. The manipulator controller 26 may additionally create certain segments (e.g., lead-in segments) when starting or resuming machining to smoothly get back to the generated tool path TP. The manipulator controller 26 may also process the virtual boundaries 71 to generate corresponding virtual constraints as described further below.

The system 10 may operate in a manual mode, such as described in U.S. Patent No. 9,119,655, incorporated herein by reference. Here, the user manually directs, and the manipulator 14 executes movement of the tool 20 and its energy applicator 24 at the surgical site. The user physically contacts the tool 20 to cause movement of the tool 20 in the manual mode. In one version, the manipulator 14 monitors forces and torques placed on the tool 20 by the user to position the tool 20. For example, the manipulator 14 may comprise the force/torque sensor S that detects the forces and torques applied by the user and generates corresponding input utilized by the control system 60 (e.g., one or more corresponding input/output signals). In some implementations, the user may be required to continually grasp a trigger or switch on the end effector to enable the force/torque sensor S that detects the forces and torques applied by the user.

The force/torque sensor S may comprise a 6-DOF force/torque transducer. The manipulator controller 26 and/or the navigation controller 36 receives the input (e.g., signals) from the force/torque sensor S. In response to the user-applied forces and torques, the manipulator 14 moves the tool 20 in a manner that emulates the movement that would have occurred based on the forces and torques applied by the user. Movement of the tool 20 in the manual mode may also be constrained in relation to the virtual boundaries 71 generated by the boundary generator 66. In some versions, measurements taken by the force/torque sensor S are transformed from a force/torque coordinate system FT of the force/torque sensor S to another coordinate system, such as a virtual mass coordinate system VM in which the virtual simulation 88 is carried out on the virtual rigid body model of the tool 20 so that the forces and torques can be virtually applied to the virtual rigid body in the virtual simulation 88 to ultimately determine how those forces and torques (among other inputs) would affect movement of the virtual rigid body, as described below.

The system 10 may also operate in a semi-autonomous or automated mode in which the manipulator 14 moves the tool 20 along the milling path 72 (e.g., the active joints J of the manipulator 14 operate to move the tool 20 without requiring force/torque on the tool 20 from the user). An example of operation in the automated mode is also described in U.S. Patent No. 9,119,655, incorporated herein by reference. In some embodiments, when the manipulator 14 operates in the automated mode, the manipulator 14 is capable of moving the tool 20 free of user applied forces. In other words, the user does not need to physically contact the tool 20 to move the tool 20. Instead, the user may use some form of remote control to control starting and stopping of movement. For example, the user may hold down a button of the remote control to start movement of the tool 20 and release the button to stop movement of the tool 20.

The system 10 may also operate in a guided-manual mode, as described in U.S Patent Application Publication No. US 2020/0281676 A1, entitled "Systems and Methods for Controlling Movement of a Surgical Tool Along a Predefined Path", the contents of which are hereby incorporated by reference in their entirety. In the guided-manual mode, the user applies forces/torques to the force/torque sensor S and the applied forces/torques are utilized to determine how far to advance the tool 20 along the tool path TP. In the guided-manual mode, the tool 20 is constrained to the tool path TP in 2DOF normal to the tool path, but unconstrained in 1DOF tangential to the tool path TP. In effect, this enables the tool 20 to freely move along the tool path TP based on manual input, but the constraints guide the user by restricting the manual movement of the tool 20 to be along the tool path.

The techniques described herein can utilize constraint equations and data, forward dynamics algorithms, rigid body calculations, constraint force calculations, and virtual simulations like those described in U.S Patent Application Publication No. US 2020/0281676 A1, entitled "Systems and Methods for Controlling Movement of a Surgical Tool Along a Predefined Path", the contents of which are hereby incorporated by reference in their entirety.

### II. Overview of Haptic Force and Joint Torque Control of Manipulator

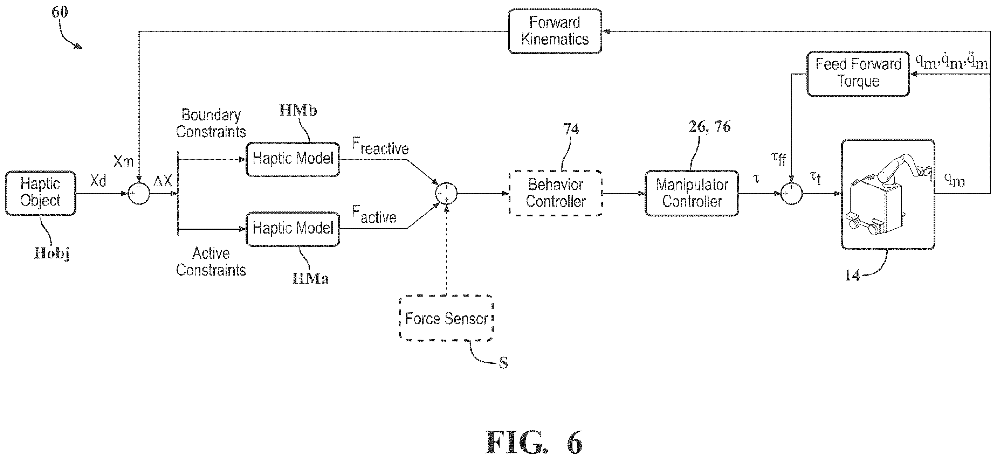

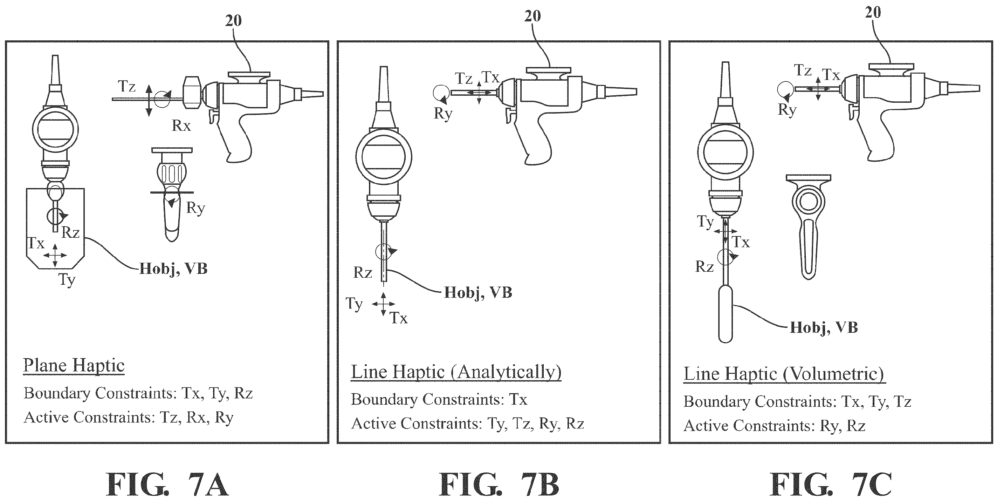

Referring to FIGS. 6 and 7, described in this section is an example control scheme implemented by the control system 60 for controlling the manipulator 14 using haptic forces and joint torques.

The control system 60 is configured to implement constraints on the manipulator 14 or the surgical tool 20 pursuant to predefined virtual fixtures or haptic objects (Hobj). Although the robot control problem varies for each type of surgical procedure or tool, these constraints can be divided into two subspaces i.e., active constraints and boundary constraints. The active and boundary constraints impose "task space" constraints on movement of the surgical tool 20. The task space is the Cartesian space defined by the task the manipulator 14 is performing. The task space can be defined by the set of all possible poses of the surgical tool 20 for the given task. The dimensions of the task space will depend on the surgical procedure, step of the procedure, type, or surgical tool 20, or the like. For example, tasks in robotics-assisted arthroplasty require up to six DOF of manipulator control depending on the type of the resection and the surgical tool 20 used for that resection. As an example, total knee arthroplasty (TKA) saw cutting can be a fully constrained task which requires the saw blade to be fully controlled in all six DOFs. Screw/post placement task in shoulder and spine surgery may require a burring attachment with robot control in five DOF (roll motion control is not required during burring). The task space for robotic surgery may be defined by a coordinate system of the implant, patient, or robot, or combinations thereof. When the manipulator 14 comprises kinematic redundancy, the described techniques can be utilized to control the manipulator 14 and/or surgical tool beyond 6DOF.

Active constraints attempt to actively virtually constrain specified DOF(s) of the surgical tool 20. Active constraints provide the surgeon with resistance or guidance of the surgical tool 20 by actively restricting a surgeon's movement of the surgical tool in a specified manner. For example, active constraints can constrain certain DOF of the surgical tool so that the surgical tool will feel stiff if the surgeon attempts to move the tool in one of the constrained DOF. Such active constraints can be imposed in any of the described operating modes of the manipulator 14, i.e., manual mode, automated mode, guided-manual mode, etc. It is not necessary that the surgeon interact with the tool 20 to impose the active constraints.

Boundary constraints are used to provide a boundary on movement of the surgical tool 20, e.g., to keep the surgical tool in a zone or keep the surgical tool out of a zone. The boundary constraints are the above-describe virtual boundaries VB that are generated by the boundary generator 66. For the boundary constraints, a reaction force is generated in response to contact or potential contact of the tool 20 to the virtual boundary VB. Boundaries can be dynamically changed during the procedure. Boundary constraints are also dynamically altered as needed throughout the procedure. For example, boundary constraints can be extended automatically or in response to certain conditions or user activation. Such boundary constraints can be imposed in any of the described operating modes of the manipulator 14, i.e., manual mode, automated mode, guided-manual mode, etc.

As shown in FIG. 6, the control system 60 is configured to implement a haptic model (HMb) for computing haptic forces to implement boundary constraints and a haptic model (HMa) for computing haptic forces to implement active constraints. These haptic models (HMa, HMb) can be separate or combined. When separate, as shown, one or more haptic object(s) (Hobj) is inputted into each haptic model (HMa, HMb). The haptic object (Hobj) may be the same or different for each model. Either of these haptic models can be implemented using any suitable control scheme, including but not limited to a spring-damper model; a proportional-derivative (PD) model; or an impulse model. The active constraint haptic model (HMa) generates haptic forces (F_{active}) that are intended to actively constrain specified DOF of the surgical tool, i.e., pursuant to the haptic object definition. The boundary constraint haptic model (HMb) generates reactive haptic forces (F_{reactive}) that are configured to reduce interaction between the surgical tool 20 and the virtual boundary VB or haptic object geometry if such interaction is present or imminent. Impulse modeling can be used to compute reactive haptic forces without requiring boundary penetration.

Referring to the examples of FIGS. 7A-7C, various examples of active and boundary constraints are illustrated with respect to the surgical tool 20. For boundary constraints, the haptic objects (Hobj) can be implemented with a variety of shapes including some predefined geometric constraints such as a plane (FIG. 7A), a line (FIG. 7B), or a volume (e.g., cylinder, cone, or box) haptic. Haptic object shape can be also defined more generically using a polygon mesh constraint which is composed of a set of triangles that are connected by their common edges and vertices. For example, in FIG. 7C, the haptic object (Hobj) for the boundary constraint is implemented as a line haptic defined by a mesh volume.

For active constraints, the haptic objects (Hobj) can be implemented by DOF restrictions on the surgical tool 20, which can mimic an intended haptic geometry. In the example of FIG. 7A, the haptic object (HobJ) for the active constraint is a haptic plane and the haptic control model (HMa) is configured to compute haptic forces (F_{active}) that are intended to constrain at least three specified DOF of the surgical tool 20 relative to the haptic plane. In this case, the at least three specified DOF comprise two rotational DOF (Rx, Ry) and at least one translational DOF (Tz). This way, the tool 20 is allowed to translate in and out of the plane (Tx), translate left or right in the plane (Ty), and rotate in within the plane (Rz), while respecting the 3DOF of the planar constraint (Tz, Rx, Ry). Such planar constraints may be suitable for constraining a saw blade during resection, for example. Notably, the planar constraint can be up to 6DOF. The planar constraint may require less DOF when the robotic system employs a passive joint or planarly extending joint.

In FIGS. 7B and 7C, the haptic object (HobJ) for the active constraint is a haptic line. The haptic control model (HMa) is configured to compute the haptic forces (F_{active}) that are intended to constrain at least four specified DOF of the surgical tool 20 relative to the haptic line. The at least four specified DOF can include at least two rotational DOF (Ry, Rz) and two translational DOF (Ty, Tz). This way, the tool 20 is allowed to translate up/down the haptic line (Tx) and rotate (about the tool axis, Rx) corresponding to the haptic line, while respecting the 4DOF of the line constraint (Ty, Tz, Ry, Rz). Similarly, the line constraint can be more restrictive, if desired, e.g., up to 6DOF. In the case in which the active constraint is implemented by a haptic volume, the haptic control model (HMa) is configured to compute haptic forces (F_{active}) intended to constrain up to three specified DOF of the surgical tool 20 relative to the haptic volume. For example, up to three rotational DOF can constrained. Notably, the planar constraint can be up to 6DOF. Such line constraints may be suitable for constraining a cutting bur, router, drill, screwdriver, or any other tool with a straight shaft, for example.

The haptic forces can be combined together to generate a total haptic force. If the described force/torque sensor (S) is utilized (as shown in FIG. 6), the force/torque values obtained from the sensor (S) can be combined with the total haptic force to generate a total force. Based on the total force, the control system 60 (or manipulator controller 26 and/or motion controller 76) is configured to generate commanded joint torques (τ) to control the respective joint(s) (J) of the manipulator 14. If the total force includes active haptic forces (F_{active}), the commanded joint torques will include components to move to constraint poses in attempt to actively constrain the specified DOF of the surgical tool. If the total force includes reactive haptic forces (F_{reactive}), the commanded joint torques will include components to alleviate tool-boundary interaction. If the total force includes forces from the force/torque sensor S, the commanded joint torques will include components to move the surgical tool 20 in a manner that mimics the surgeon's interaction with the surgical tool 20, while respecting the active and boundary constraints. This type of computation can be part of an admittance type system that optionally utilizes the behavior controller 74 to implement a virtual rigid body simulation of the total force to determine commanded positions or joint torques.

The computed joint torque (τ) pursuant to the haptic model calculations may optionally be combined with additional feed forward torques (τ_{ff}). Feed forward torques can include gravity torques and forces required for joints to maintain their positions in the specified gravity. Gravity torques and forces can be computed from the actual measured joint positions (qₘ). Feed forward torques can also include joint damping torques to smooth out movements and prevent oscillations of the joints. Damping torques can be calculated from the velocity (q dot m) of the joints. The feed forward torques (τ_{ff}), and the commanded joint torques (τ) from the haptic constraints can be combined into a total commanded torque (τₜ) by which the control system 60 will command the joints. Forward kinematic calculations can be used to determine the measured pose (Xₘ) of the surgical tool 20 or TCP. The measured pose (Xₘ) of the surgical tool 20 can be fed back into the control loop to determine the difference between the measured pose (Xₘ) and the desired pose (X_{d}) of the surgical tool relative to the haptic objects.

The difference of this comparison (ΔX) is used in the next time step to re-compute the necessary boundary and active constraints, and this process can repeat for multiple time steps. The processes described herein can be iteratively performed and repeated for any number of time steps during run-time of the manipulator 14 and can do so depending on presence or absence of conditions, such as detected tool-tissue interaction, surgical steps, operation of certain modes of operation (e.g., manual, automated, guided-manual), etc. This way, the machine learning models (MLM) can continue adaptive learning and optimization of the robotic behavior.

**III.** Techniques for Employing Machine Learning Models to Improve Surgical Robotic Performance

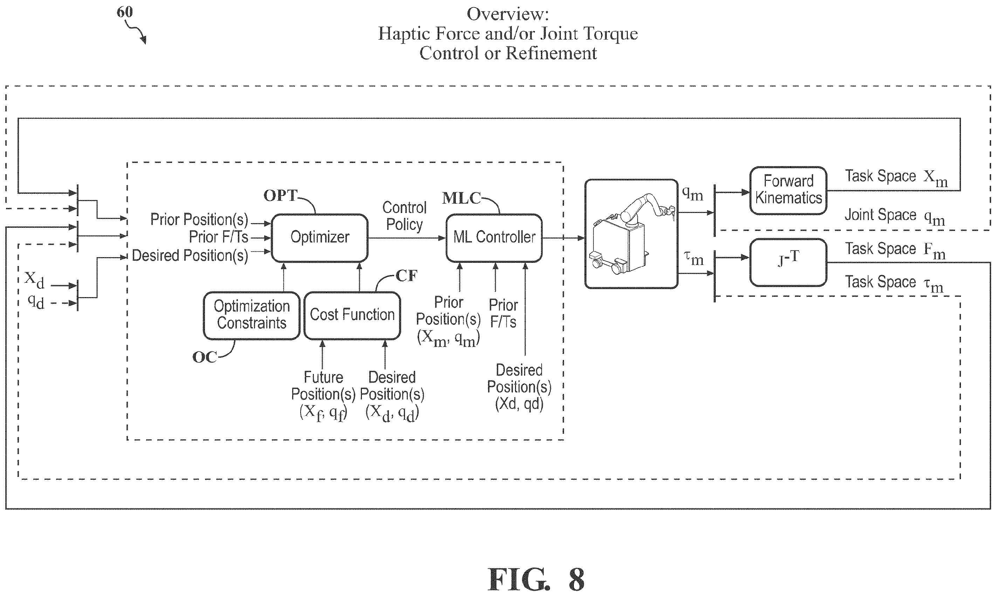

With reference to FIGS. 8-19, described herein are systems, methods, and non-transitory computer readable media (computer program products) for employing machine learning models to improve performance and accuracy of the manipulator 14 and/or surgical tool 20. The described techniques can improve accuracy of the surgical tool 20 while the tool 20 interacts with the surgical site and can provide the high level of tool accuracy needed for robotic surgery. As a result, improved robot/tool accuracies reduce disturbances to the surgeon and complications during surgery thereby potentially improving the surgical outcome for the patient. The described solutions can further compensate the impact of surgeon disturbances (misuse of the robot by applying extra forces or applying accidental forces to robot links) during the procedure. The techniques described herein are particularly suited to compensate for complex non-linear errors thereby reducing steady state errors affecting robot/tool accuracy. The non-linear errors can arise from many sources, such as but not limited to variant inertia, friction, joint flexibility, backlash, external disturbances, tool interactions, and boundary variations (corners, edges, overlapping boundaries, changing boundaries). The described solutions can eliminate steady state error, unwanted tool vibrations, and/or tool inaccuracies in a manner that is more intelligent, predictive, and faster than existing control schemes.

### A. Overview of Machine Learning Control Scheme

Described herein are solutions to compensate for non-linear errors in the task space and/or in the joint space of the manipulator 14. For example, the described control schemes can generate refinements to computed haptic forces and/or to computed joint torques. Refinements to haptic forces can be made in attempt to achieve or maintain a desired pose of the surgical tool 20 according to specified DOF defined by active constraints, thereby providing a more accurate and stiff active constraint. Refinements to joint torques can be made in attempt to achieve or maintain the desired joint positions for the joints of the manipulator 14, thereby providing more accurate and stiff joint response. By generating refinements to computed forces/torques, the machine learning model schemes described herein can be seamlessly integrated into robotic control schemes to improve accuracy without requiring substantial reconstruction of the control system 60. The joint space can be defined by the set of all possible positions that the joints (J) of the manipulator 14 can achieve given the kinematic configuration of the links and joints.

Although the task space and joint space control schemes are introduced together in this section, the two spaces can be related using Jacobian transformation. Therefore, it should be understood that the control system 60 can be configured to perform the described control/refinement of the task space and/or joint space separately or in combination. In other implementations, the described solutions can (fully or partially) compute haptic forces and/or computed joint torques, instead of refining the same.

With reference to FIG. 8, an example block diagram is provided of control processes that can be performed by the described techniques for compensating for errors, in either, or both the task space and/or in the joint space of the manipulator 14. Throughout this description, the steps or processes of the control schemes will be described as being performed by the control system 60. As described, the control system 60 can include any one or more of the described controllers or components of the surgical system. One or more machine learning models (MLM) can be added or incorporated to the control system 60 to refine aspects of the control system 60. In other implementations, the machine learning model(s) (MLM) can used as a substitute for components/software of the control system 60. For example, the machine learning model(s) (MLM) can substitute for the haptic control models (HMa, HMb), behavior controller 74, and/or the motion controller 76. In other instances, the machine learning model(s) (MLM) can be used to control the entire 6DOF motion of the tool 20 or be used to control or constrain specific DOF or a motion task of the tool 20. Moreover, in some variations, the machine learning model(s) (MLM) may be configured to control the manipulator 14 position with or without being used to control the tool position.

Notably, using the machine learning model(s) (MLM), the techniques described herein can provide "data-driven" solutions to robotic control. By data-driven, it is understood that the model for refining control of the manipulator 14 can be derived from real-world experiences of the manipulator 14 and/or surgical tool 20, without necessarily requiring a complete pre-programmed control model. The data-driven techniques can receive large amounts of prior (position/force) measurements of the manipulator 14 to dynamically derive a control model and evolve the control model over time to effectively learn optimal behavior for the manipulator 14 and/or tool 20 given certain conditions. In some cases, the data-driven techniques can be individually tuned/trained or learn optimal behavior for specific robotic behaviors or surgical tasks, such as, but not limited to drilling a hole, cutting a plane, or adapting to the specific behaviors of the surgeon. The data-driven control model can adapt to compensate for unexpected situations and non-linearities. Data-driven techniques described herein can include but are not limited to model-free adaptive control (MFAC), model-free predictive control (MFPC), deep learning, reinforcement learning, deep reinforcement learning, integral reinforcement learning, imitation learning, or the like. It is also contemplated to utilize a novel approach to pure data-driven control using a variation of MFAC or MFPC that employs the described neural network(s). Although data-driven models are contemplated, it should be noted that the described techniques may be used with pre-programmed models and the solutions are not necessarily limited to data-driven control.

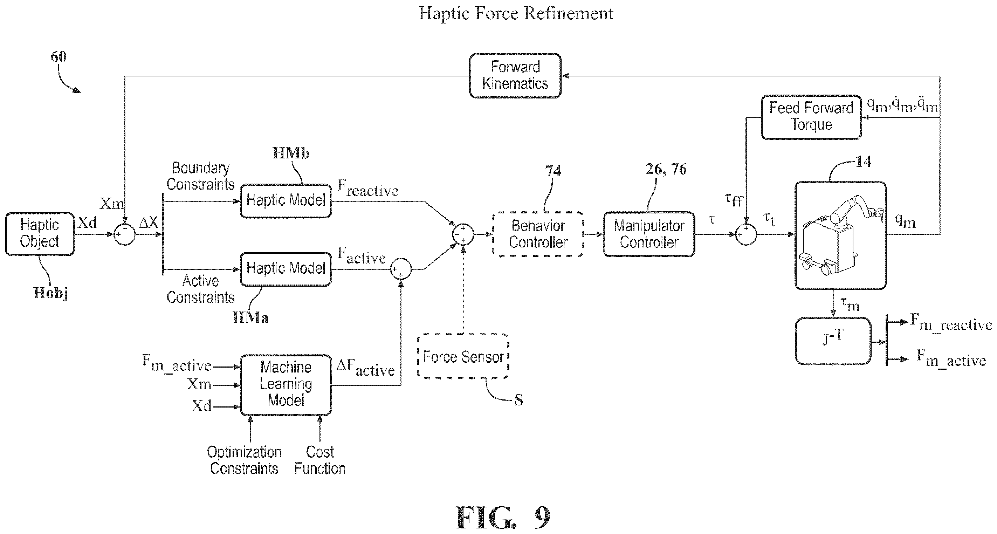

As shown in the diagram and will be described below, the machine learning model (MLM) is configured to receive and process various input values, including prior poses/positions, prior forces/torques (F/T), and a desired value. The prior poses/positions are previously (measured) poses or positions of components of the manipulator 14. The prior forces/torques (F/T) are previously (measured) forces or torques generated by components of the manipulator 14. As it relates to these values, the term "prior" is synonymous with "measured" and the following description may use these terms interchangeably. The desired value is one that the control system 60 may use as a target, reference, or optimal value for how to control one or more components of the manipulator 14. Each of these inputs will be described below. Moreover, the sections below will describe various examples of implementing the machine learning model (MLM) for joint space and/or task space control (e.g., FIGS. 9-18) Any of the inputs, outputs, and/or architecture of the (MLM) described in this section can be fully applied to the various examples described below.

In one implementation, the prior poses/positions are related to the task space of the manipulator 14. Here, the prior poses/positions can include previously measured poses (Xₘ) of the surgical tool 20. In some instances, the previously measured tool poses (Xₘ) of the surgical tool 20 are constraint poses. Constraint poses are poses to which the surgical tool 20 was commanded in attempt to satisfy a constraint imposed by the control system 60. For example, the control system 60 can compute haptic forces that are intended to constrain specified DOF of the surgical tool 20. Based on the haptic forces, the control system 60 can generate commanded joint torques to control the manipulator 14 to move to constraint poses in attempt to actively constrain the specified DOF of the surgical tool 20. Any number of N previous samples of the tool poses can be acquired. The prior tool pose(s) can be derived by obtaining prior measured joint positions/angles (qₘ) of the joints (J) of the manipulator 14 and applying the measured joint positions/angles to a forward kinematics model of the manipulator 14 or by otherwise using Jacobian transforms from joint position to tool position. Other sensing means, such as tracking data of the tool 20 derived from the navigation system 32 or inertial or motion sensors on the surgical tool 20 can be utilized to determine the measured poses (Xₘ).

Additionally, or alternatively, the prior poses/positions can be related to the joint space of the manipulator 14. For example, prior positions can include prior joint positions/angles (qₘ) that were measured at the joint(s) of the manipulator 14 after application of the computed joint torques (τ, τₜ) for the respective joint(s). The control system 60 controls the joints of the manipulator 14 in accordance with the computed joint torques to move the joints (J) to their respective joint positions/angles (qₘ). In some cases, prior joint positions/angles (qₘ) can be measured responsive to the manipulator 14 imposing a constraint pose on the surgical tool 20. However, this need not always be the case because the prior joint positions/angles (qₘ) can be based on any other commanded movement of the manipulator 14. Any number of N previous samples of the joint positions can be acquired. The prior joint positions/angles (qₘ) can be measured using any suitable means, such as joint encoders, kinematic analysis, potentiometers, inertial sensors, navigation system-based tracking, machine vision tracking, or the like.

Regarding the prior forces/torques (F/T), these can be related to the task space of the manipulator 14. In one implementation, the prior forces/torques (F/T) include previously measured forces acting on the surgical tool 20. Such forces may be measured at, or otherwise derived from movement of, the surgical tool 20. Such measured forces can include components of forces commanded on the surgical tool 20 to constrain the surgical tool 20 relative to virtual boundary VB and/or to actively constrain the surgical tool 20 using the described active constraints. Importantly, the measured tool forces can also include force components derived from (usually unknown) external disturbance(s) acting on the surgical tool 20 or manipulator 14, such as from tool-tissue interaction, collisions, human interaction, and the like. Any number of N previous samples of the forces acting on the tool can be acquired. In one example, the measured tool forces are derived by converting actual (measured) joint torques (τₘ) generated by the actuators of the active joints (J) of the manipulator 14 during prior movements of the surgical tool 20. Actual joint torques are generated responsive to commanded joint torques (τₜ) applied to the manipulator 14. The measured joint torques can be converted into forces using an inverse Jacobian transformation. Measured forces can also be derived by converting electrical current draw of active actuators of the manipulator 14 into forces. Other forms of force sensing can be utilized, such as by implementing other force or pressure sensors, or by implementing a force observer to indirectly infer forces acting on the tool 20 using indirect parameters such as velocity and/or acceleration. The force sensor(s) can be external to the manipulator 14 and need not necessarily be located on the manipulator 14.

Additionally, or alternatively, the prior forces/torques (F/T) can be related to the joint space of the manipulator 14 and can include previously (measured) joint torques. Previously measured joint torques may be torques measured at, or otherwise derived from movement of, one or more active joints (J) of the manipulator 14. Such measured joint torques can represent torques applied by the joints (J) in attempt to move the joints (J) to commanded joint positions, e.g., to move the surgical tool 20. Importantly, the measured joint torques can similarly include torque components derived from (usually unknown) external disturbance(s) acting on the joints (J). Such external disturbance(s) can be indirectly imparted on the joint(s) based on disturbances acting on the surgical tool 20, such as from tool-tissue interaction, collisions, human interaction, and the like. Any number of N previous samples of the joint torques can be acquired. Measured joint torques are generated responsive to commanded joint torques (τₜ) applied to the manipulator 14. Prior joint torques can be derived by measuring electrical current draw of active actuators of the manipulator 14. Other forms of torque sensing can be utilized, such as by implementing other torque sensors, or by implementing a torque observer to indirectly infer torques acting on the joint actuators using indirect parameters such as joint velocity and/or acceleration.

Another input into the machine learning model (MLM) is target, reference, or desired value(s) (Xₐ, q_{d}). The desired value(s) (Xₐ, q_{d}) can be predetermined, generated, inferred, or predicted by the control system 60 or machine learning model (MLM). The desired value(s) (X_{d}, q_{d}) can represent a desired behavior of the manipulator 14 and/or surgical tool 20. In one example, the desired value can be a desired pose or position of components of the manipulator 14 and/or surgical tool 20. For instance, the desired value can be one or more desired pose(s) (X_{d}) for the surgical tool 20. In one example, the desired pose can be desired constraint pose(s) or the ideal pose(s) at which the surgical tool 20 should be placed to maintain the constraint of specified DOF of the surgical tool. The desired constraint pose will differ depending on the nature of constraint or configuration of the surgical tool 20. In another example, the desired value can be desired joint positions (q_{d}) for one or more joint(s) of the manipulator 14. The desired joint positions (q_{d}) can be positions necessary to achieve the constraint pose of the surgical tool 20. In other examples, the desired joint positions (q_{d}) can be positions that are desired for any other purpose, such as eliminating or reducing non-linear or steady state errors. Depending on the type of surgical tool 20 or surgical procedure, for example, the desired values (Xₐ, q_{d}) can be defined for any other suitable purpose.

The machine learning model (MLM) is also configured to receive future values as an input. Future values can be values that predict or represent any future or expected behavior of any one or more components of the manipulator 14 and/or surgical tool 20. The future behavior may be ideal or may be sub-optimal (trial and error). In one example, the future values can be future poses or positions of components of the manipulator 14 and/or surgical tool 20. For instance, the future values can be one or more future pose(s) (X_{f}) for the surgical tool 20. In one example, the future pose can be a future constraint pose(s) at which the surgical tool 20 may be placed to maintain the constraint of specified DOF of the surgical tool. The future constraint pose values may differ depending on the nature of constraint or configuration of the surgical tool 20. In another example, the future values can be future joint positions (q_{f}) for one or more joint(s) of the manipulator 14. The future joint positions (q_{f}) can be future positions necessary to achieve the constraint pose of the surgical tool 20. In other examples, the future joint positions (q_{f}) can be future positions for any other purpose, such as eliminating or reducing non-linear or steady state errors. Depending on the type of surgical tool 20 or surgical procedure, for example, the future values (X_{f}, q_{f}) can be defined for any other suitable purpose.

The future values (X_{f}, q_{f}) can be predetermined, generated, inferred, or predicted by the control system 60 or machine learning model (MLM). In some cases, future values can be derived from any of the prior (measured) position/pose values (Xₘ, qₘ). For example, the control system 60 and/or machine learning model (MLM) can employ any suitable algorithm or modeling to determine the future values (X_{f}, q_{f}). Such algorithms or modeling may include regression models, neural networks, clustering models, or the like.

Having introduced various inputs into the machine learning model (MLM), we now introduce components/modules/features of the machine learning model (MLM) that will process the inputs. The features of the machine learning model (MLM) can be implemented by any one or more components (e.g., controllers, processors, memory) of the control system 60. In some cases, it is contemplated that the machine learning model (MLM) can partially or fully be implemented in a remote controller or remote server that is remotely coupled to the manipulator 14. The remote controller/server can form part of the control system 60. For example, the described inputs can be transmitted over the internet to the server for receipt by the machine learning model (MLM). The machine learning model (MLM) can remotely transmit control signals and/or refinements over the internet to the manipulator 14 or control system 60.

With continued reference to FIG. 8, the control system 60 and/or machine learning model (MLM) are configured to implement, process, and/or utilize a cost function (CF), an optimizer (OPT), optimization constraints (OC), and a machine learning controller (MLC). Each of these features will be described below.

The cost function (CF) is a mathematical formula that is defined as a metric for the optimizer (OPT) to derive the policy (or refinement policy) update such that it minimizes the cost function (CF). It can be also defined to evaluate the machine learning model (MLM) identification/prediction and to derive a policy update for the neural network (NNopt) to increase the accuracy of the future identification/prediction. The future position/pose value(s) (X_{f}, q_{f}) and desired position/pose value(s) (Xₐ, q_{d}) are inputted into the cost function (CF). Effectively, the cost function (CF) can be used to measure differences between the future position/pose value(s) (X_{f}, q_{f}) and the desired position/pose value(s). The machine learning model (MLM) optimizes (e.g., minimizes) the cost function (CF) to obtain a policy update law. For example, using the cost function (CF), the machine learning model (MLM) can minimize one or more of the following: future residual errors, oscillation in constraint poses, and/or energy injected into the manipulator 14. To account for non-linearities, the cost function (CF) can be formulated as a non-quadratic equation.

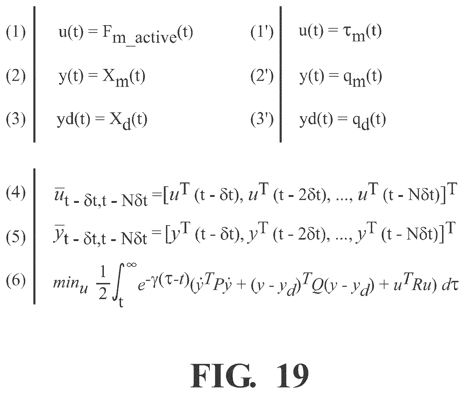

One example formula for the cost function (CF) is shown at equation (6) of FIG. 19. In equation (6), the future residual errors are denoted by the expression (*y-y_{d}*)*^{T} Q*(*y-y_{d}*), the oscillation in constraint poses is denoted by the expression (*ẏ^{T}Pẏ*) and the amount of energy injected into the manipulator is denoted by the expression (*u^{T}Ru*)*.* The model error measures how accurately the machine learning model (MLM) was able to predict relationships between the future position/pose value(s) (X_{f}, q_{f}) and desired position/pose value(s) (X_{d}, q_{d}). It also indicates how the machine learning model (MLM) was successful in applying the appropriate refinement for the given dynamical changes in the system. The model error output of the cost function (CF) can be used to adjust or optimize parameters of the machine learning model (MLM) in attempt to facilitate training of the (MLM) for minimizing future errors. Depending on the input, the model error outputted by the cost function (CF) can represent different adjustments or optimizations. The machine learning model (MLM) can be iteratively optimized based on the output of the cost function using any suitable optimization algorithm, such as gradient descent.

The optimizer (OPT) is configured to receive the prior (measured) position/pose value(s) (Xₘ, qₘ), the prior (measured) force/torque value(s), and the desired position/pose value(s) (Xₐ, q_{d}). The optimizer (OPT) also receives the model error outputted by the cost function (CF). The optimizer (OPT) can also be subjected to optimization constraints (OC). One objective of the optimizer (OPT) is to explore or define control policies that optimize the cost function (CF) and are based on the prior incoming data. The control policies will be used as a strategy to control or refine actions of the manipulator 14. The optimizer (OPT) can adaptively identify control models, adaptively predict control models, or implement an adaptive critic model to evaluate actions.

The optimization constraints (OC) can be imposed on the control signals that are inputs to the robotic processes (hard constraints) and/or the output of the controlled robotic processes (performance constraints). For example, the optimization constraints (OC) can define constraints on prior, future, or desired position/pose value(s) and/or force/torque value(s). The optimization constraints (OC) can also impose constraints on states of the manipulator 14 and/or surgical tool 20. Due to the desire to account for non-linearities, the optimization constraints (OC) can be defined as non-linear constraints. Example optimization constraints (OC) can include, but are not limited to overshoot constraints, band constraints, actuator non-linearity constraints, surgical tool non-linearity constraints, nonminimal phase behavior constraints, joint limits, velocity bounds, workspace boundaries, or the like. In one example, optimization constraints (OC) apply a range defining a minimum value and a maximum value for the haptic force and/or joint torque refinement. The control system can apply the refinement only in response to the refinement falling within the range. As such, the optimization constraints (OC) can be used to limit the amount of force/torque refinement, e.g., to avoid oversaturation or provide predictable response. The optimization constraints (OC) need not always be applied. The optimization constraints (OC) can be become "active" in certain conditions (e.g., when non-linear errors arise).

The optimizer (OPT) processes the prior position/pose value(s) (Xₘ, qₘ), the prior force/torque value(s) (F/T), and the desired position/pose value(s) (X_{d}, q_{d}), while optionally being subject to optimization constraints (OC). The optimizer (OPT) is configured to generate a control policy. The control policy can be iteratively adapted or optimized by the model error outputted by the cost function (CF). The control policy defines the decision-making strategy, rules or heuristics that are utilized to select actions for the controlling components of the manipulator 14. In one example, the control policy is specifically a refinement control policy, which defines the rules used to refine the haptic forces and/or joint torques. One goal of the optimizer (OPT) is to determine the most optimal control policy to achieve a desired outcome. For the refinement control policy, the desired outcome may be, for example, to achieve the desired pose of the surgical tool 20 or the desired positions of the joints (J). When the machine learning model (MLM) is used to directly control the manipulator 14 (rather than to refine force/torque values), the control policy may be defined to obtain any other desired outcome such as, for example, to achieve the desired behavior of the surgical tool 20 and/or the desired behavior of the joints (J). Through the described process of obtaining and assessing prior values, the control policy, or refinement control policy, is iteratively updated as the machine learning model (MLM) continues learning from environmental conditions.

As will be described in the examples below, the optimizer (OPT) can take various configurations or forms depending on the nature of the machine learning model (MLM). As will be described below, the optimizer (OPT) can be included in various architectures, such as, but not limited to model-free adaptive control (MFAC), model-free predictive control (MFPC), and deep refinement learning (DRL). In one example, the optimizer (OPT) can include a neural network (NNopt) that is responsible, in part, or in whole, for defining the control policy. In some cases, the neural network (NNopt) can receive, at the input layer, the prior position/pose value(s) and the prior force/torque value(s), or variations thereof. In other instances, the neural network (NNopt) can additionally receive the desired position/pose value(s) (X_{d}, q_{d}) at the input layer. The neural network (NNopt) can include any suitable number of hidden layers and can be any suitable type of neural network architecture, including but not limited to: deep-learning neural network, a deep-reinforcement learning neural network, a recurrent neural network (RNN), a multilayer perceptron (MLP), a feed-forward neural network, a convolutional neural network (CNN), or the like.

The output layer of the neural network (NNopt) can produce various results depending on the network configuration. In one example, the output layer can produce the control policy or an update law that is used to modify the control policy. In another example, the output layer produces an identification of a state of the manipulator 14 or a prediction/estimate of the state of the manipulator 14. The identified or predicted state values can be subjected to optimization using the cost function (CF) and/or optimization constraints (OC) to define the control policy.

The machine learning controller (MLC) utilizes the control policy generated by the optimizer (OPT). The machine learning controller (MLC) may be incorporated into the optimizer (OPT) or may be implemented separately therefrom. The machine learning controller (MLC) receives as an input the prior position/pose value(s) (X_{d}, q_{d}), the prior force/torque value(s), and desired position/pose value(s) (Xₐ, q_{d}). These prior and desired value(s) can be applied to the control policy generated by the optimizer (OPT) in order to determine the refinement on haptic force or joint torque. In other instances, the machine learning controller (MLC) can contribute to determining or refining the control policy or generating a separate control policy for robotic actions.

As will be described in the examples below, the machine learning controller (MLC) can take various configurations or forms depending on the nature of the machine learning model (MLM). The machine learning controller (MLC) can be included in various architectures, such as, but not limited to model-free adaptive control (MFAC), model-free predictive control (MFPC), and deep refinement learning (DRL). The machine learning controller (MLC) may be a linear controller or a non-linear controller and may be configured to apply adaptive control, predictive control, or implement reinforcement learning. For example, the machine learning controller (MLC) can implement model adaptive control to iteratively and dynamically modify the gains of the control policy by comparing the real-time identified state of the manipulator 14 with the desired pose/position value(s), and optionally, the prior values. The machine learning controller (MLC) can implement model predictive control (MPC) to predict future states of the manipulator 14 and determine robotic control actions that minimize the cost function (CF) over a finite horizon. In other instances, the machine learning controller (MLC) can be adapted to learn from past experiences to optimize the action control strategy. For example, the machine learning controller (MLC) can include a neural network (NNmlc) that is responsible, in part, or in whole, for developing a separate control policy on actions for the manipulator 14. The neural network (NNmlc) can include any suitable number of hidden layers and can be any suitable type of neural network architecture, including but not limited to: deep-learning neural network, a deep-reinforcement learning neural network, a recurrent neural network (RNN), a multilayer perceptron (MLP), a feed-forward neural network, a convolutional neural network (CNN), or the like.

Using any of the described techniques, the machine learning controller (MLC) outputs control values or refined control values, for computation of haptic force/joint torque. The manipulator controller 26 can process the control values or refined control values for controlling the manipulator 14. For example, the refinement of the haptic forces is utilized to control the manipulator 14 to move to refined constraint poses in attempt to achieve the desired pose of the surgical tool 20 according to the specified DOF. The haptic forces include a haptic force component for each of the specified DOF. The refinement can include a force refinement to be added to the haptic force component for each of the specified DOF. Similarly, the refinement of the joint torques is utilized to control the manipulator 14 in attempt to move the joints (J) to the desired joint angles. The computed joint torques can include a torque value for each active joint of the manipulator 14. Here, the refinement can include a torque refinement to be added to the computed torque value for each active joint of the manipulator 14. The control system 60 can collect prior pose/position values and prior force/torque values after execution of each refined action. The process can repeat for subsequent time steps, and so on.

Notably, the described machine learning models (MLM) can be configured to be limited in their use, e.g., for safety purposes or regulatory compliance. For instance, the described machine learning models (MLM) may be intentionally limited to specific motions or tasks or used only during the presence of certain conditions or during specified times. The control system can maintain the (standard) haptic and joint control schemes in the event that the machine learning model (MLM) is temporarily deactivated or turned off. The timing and conditions of activation of the described machine learning models (MLM) can be controlled or scheduled by a human (e.g., surgeon, staff, robotic designer) or can be automatically regulated by the control system.

### B. Haptic Force Refinement

Referring to FIGS. 9-13, we now describe examples of the control system 60 implementing the various machine learning models (MLM) to improve performance of active constraints by injecting a refinement (ΔF_{active}) to the haptic forces (F_{active}) in attempt to more stiffly and accurately constrain specified DOF of the surgical tool.

To further elaborate, due in part to unforeseen disturbances and non-linear errors, the active constraints may not be able to perfectly constrain the specified DOF of the surgical tool 20. This may result in tool 20 vibrations or oscillations as the control system 60 attempts to satisfy the constraints and minimize steady state error. Such errors may result in diminished accuracy of the surgical resection or anatomical manipulation. For this reason, we have described that the active constraints "attempt" to constrain the certain degrees DOF of the tool 20, based on the practical reality that the constraints may not always necessarily constrain the tool 20 as commanded. Although the described solutions may be used for any surgical procedure, total knee arthroplasty particularly suffers from this challenge. TKA planar cuts require the saw blade to remain on the defined cutting plane without any orthogonal deviation from the plane. With an inaccurate cutting due to the failure of maintaining active constraints successfully, the cut may be either proud or deep. If proud, the surgeon must complete the cut manually. If deep, the resection may not fit the implant. Since multiple cuts are required for the TKA application, this will result in a form error when the surgeon tries to place the implant component on the multiple cuts prepared on the bone (intercut error). This will leave the surgeon with no alternative other than cementing the implant to the bone. The solutions described herein provide control schemes designed to address the above challenges, compensate for non-linear errors, and provide a stiffer and more predictable response for active constraint implementation, thereby improving overall tool pose and/or cutting accuracy.

Moreover, it is contemplated that the described solutions can refine or regulate certain DOF, while maintaining the remaining DOF to be controlled according to the standard haptic control scheme. The selection of which DOF are controlled/refined by the machine learning model (MLM) and which DOF remain controlled according to the standard haptic control scheme can be manually specified or automatically regulated by the control system.

Figure 9 provides the control scheme of FIG. 6 modified by inclusion of the machine learning model (MLM) for haptic force refinement. To improve active constraint performance, the machine learning model (MLM) is injected into the active constraint pathway. The machine learning model (MLM) is configured to output a refinement (ΔF_{active}) on the haptic forces, which is combined with the haptic forces (F_{active}) computed by the active constraint haptic model (HMa). To provide this refinement, the machine learning model (MLM) takes many of measured inputs described in the preceding section. Namely, one input is the prior forces (F_{m-active}) acting on the surgical tool 20. As shown in FIG. 9, the prior forces (F_{m-active}) can be derived from performing an inverse Jacobian transformation (J^{-T}) on measured joint torques (τₘ). Such forces may be measured at, or otherwise derived from movement of, the surgical tool 20. Notably, the prior forces (F_{m-active}) can include not only components of haptic forces (F_{active}) but also force components derived from external disturbance(s) acting on the surgical tool 20 or manipulator 14, such as from tool-tissue interaction, collisions, human interaction, and the like. These external disturbances are usually the source of non-linear errors. Other inputs include prior (measured) constraint poses (Xₘ) of the surgical tool 20 and the desired constraint pose(s) (X_{d}) of the surgical tool 20. In addition to the measured inputs, the machine learning model (MLM) is also configured to utilize the optimization constraints (OC) and cost function (CF), which have been described above. Although the example in FIG. 9 illustrates supplementing the haptic control model (HMa) with the machine learning model (MLM), we reiterate that in an alternative implementation the machine learning model (MLM) can substitute partially or completely for the haptic control model (HMa). In other words, the machine learning model (MLM) may be configured to compute the haptic forces (F_{active}), including the refinement thereof.

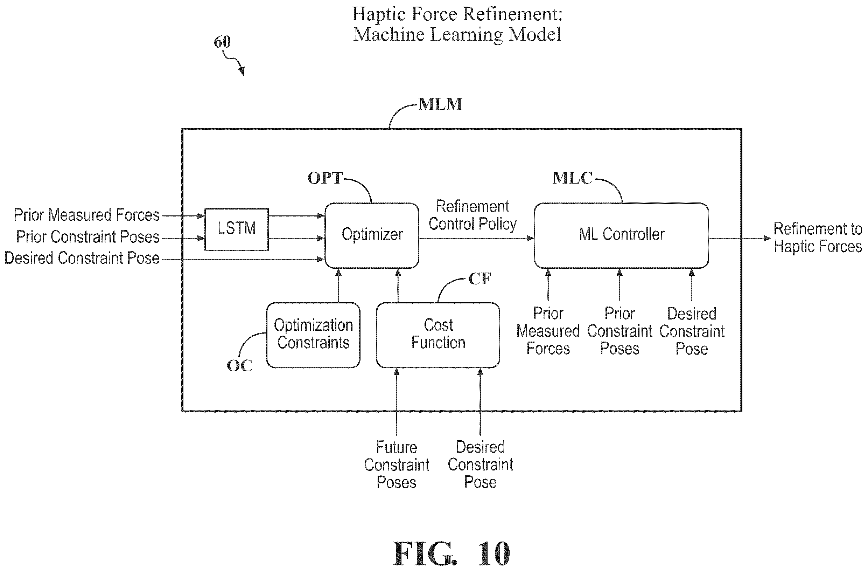

The diagram of FIG. 10 illustrates example architecture of the machine learning model (MLM) adapted for refining active constraint computation. The machine learning model (MLM) implements the optimizer (OPT) to receive the input values, i.e., prior pose value(s) (Xₘ), the prior force value(s) (F_{m-active}), and the desired pose value(s) (X_{d}). In one example, the prior pose and force values are directly inputted into the optimizer (OPT). In another implementation, the prior pose and force values are optionally pre-processed by a long short-term memory (LSTM) prior to being inputted into the optimizer (OPT). The LSTM can be part of, separate from, or coupled to an input of, the machine learning model (MLM). The LSTM provides a dynamic memory cell for the vast amount of incoming measurement data. Using the LSTM, the machine learning model (MLM) can be injected with a mini-batch of historical time-delay data, including prior haptic forces and prior constraint poses. The LSTM can include a recurrent neural network (RNN) that processes the prior pose and force values and modifies weights and biases of the RNN to learn long-term dependencies among the values and to selectively retain or discard the values. The RNN can be trained in a supervised or unsupervised fashion using sample prior pose and force measurements and by utilizing any suitable optimization algorithm, such as gradient descent, backpropagation through time (BPTT), or the like.

The future pose value(s) (X_{f}) and desired constraint pose value(s) (X_{d}) are inputted into the cost function (CF). The optimizer (OPT) also receives the model error outputted by the cost function (CF). The optimizer (OPT) can also be subjected to optimization constraints (OC). The optimizer (OPT) processes the prior constraint pose and force value(s) and desired constraint pose while optionally being subject to optimization constraints (OC). Based on the incoming data, the optimizer (OPT) defines the refinement control policy that optimizes the cost function (CF) and that defines the rules used to refine the haptic forces. One goal of the optimizer (OPT) is to determine the most optimal refinement control policy to achieve the desired pose of the surgical tool 20. The machine learning controller (MLC) can take as an input the prior force value(s) and the prior constraint pose value(s). In some cases, the machine learning controller (MLC) optionally can take in the desired constraint pose value(s). These prior and desired value(s) can be applied to the refinement control policy generated by the optimizer (OPT) in order to determine the refinement on haptic force. The refinement of the haptic forces is utilized to control the manipulator 14 to move to refined constraint poses in attempt to achieve the desired pose of the surgical tool 20 according to the specified DOF. The haptic forces include a haptic force component for each of the specified DOF. The refinement can include a force refinement to be added to the haptic force component for each of the specified DOF.

Figure 19 provides example equations and expressions (1)-(6) that the control system 60 can utilize in haptic force refinement calculations for any of the implementations described herein. Equation (1) expresses an output vector u(t) of the prior force value(s) (F_{m-active}) as a function of time. Equation (2) expresses an output vector y(t) of the prior pose value(s) (Xₘ) as a function of time. Equation (3) expresses a vector yd(t) of the desired pose value(s) (X_{d}) as a function of time. Equation (4) represents N samples of the output vector of the prior force value(s) (u(t)), where u^{T} is the transpose of the vector u(t). Equation (5) represents N samples of the output vector of the prior pose value(s) (y(t)), where y^{T} is the transpose of the vector y(t). These equations and expressions are provided as examples and are not intended to limit the scope of the described solutions.

As will be described in the following examples, the machine learning model (MLM) adapted for haptic force refinement will be described using various architectures, such as, but not limited to: model-free adaptive control (MFAC), model-free predictive control (MFPC), and deep refinement learning (DRL), and equivalents or combinations thereof. Any of these machine learning frameworks may be realized as a data-driven system. These machine-learning based frameworks aim at learning what is the best control policy for the given circumstances based on the behavior learned from the environment and understanding how the environment responds to certain haptic control policies. The diagrams of FIG. 9 and 10 can be understood to as a functional generalization that can embody any of the described examples. The proposed solutions are not limited to any particular example described herein and may be captured more as generally set forth.

Although the examples described in this section primarily focus on controlling or refining the haptic forces, it should be understood that the described control schemes may be applied to control or refine either or both of the active constraints and/or the boundary constraints, described above. Hence, any description below regarding active constraints may be substituted for boundary constraints.

### 1. Model- Free Adaptive Control for Haptic Force Refinement

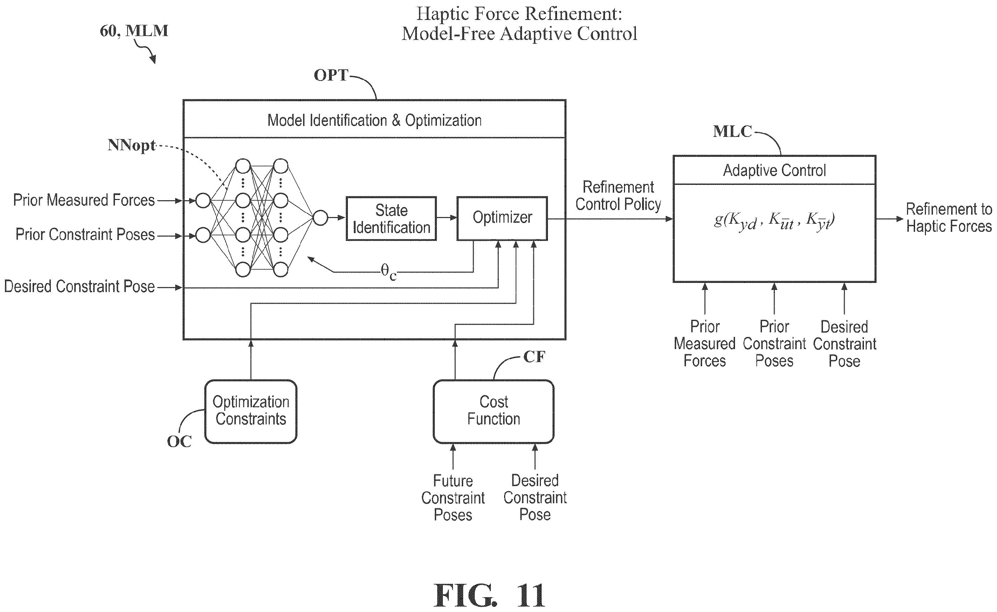

In one implementation, and with reference to FIG. 11, the machine learning model (MLM) is realized using a form of model-free adaptive control (MFAC) to generate the haptic force refinement. Model-free adaptive control enables the control system 60 to adjust the haptic refinement response dynamically, and in real-time, without requiring a detailed mathematical model of the state of the manipulator 14 or the environment in which the manipulator 14 interacts. As such, MFAC can be implemented as a data-driven control method. MFAC is able to adaptively and autonomously learn, without requiring complicated trial and error tuning procedures. MFAC is also mathematically proven to provide stability to a closed loop system. MFAC also can operate using limited input data, e.g., the prior forces and constraint poses. Using MFAC, the machine learning model (MLM) can reconstruct the states of the manipulator 14 from the measured input data and the control policy can be designed based on the linear or non-linear state feedback control theory.

Referring to FIG. 11, one example of MFAC is illustrated wherein the optimizer (OPT) is realized as a model identification and optimization block and the machine learning controller (MLC) is realized as an adaptive controller. In this example, the optimizer (OPT) is implemented with a neural network (NNopt), such as a deep-learning network. The neural network (NNopt) can receive, at the input layer, the prior forces and constraint poses, or variations thereof. The optimizer (OPT) also receives the desired constraint poses, although these values are not required to be inputted into the neural network. The neural network (NNopt) processes the prior measurements to generate an output that comprises an identification of one or more state(s) of the manipulator 14 (as shown at the "state identification" block).

Notably, the identified state of the manipulator 14 may be identified in the presence of the described non-linearities. The state of the manipulator 14 in this sense can be a state that is not otherwise easily identified or predicted based on the prior values and can be used as a surrogate to a predetermined system model of the manipulator 14. Although prior values are utilized, the identified state of the manipulator 14 are generated to account for changing conditions arising from uncertainties or non-linearities in the task space. In one example, the state of the manipulator identifies an actual or next tool pose, or an actual or next constraint pose for the tool. This state identification can be then utilized to construct a more efficient control policy when the manipulator 14 dynamics are partially known (grey box) or fully unknown (black box).

The optimizer (OPT) can automatically identify, generate, or update the control policy. Namely, the identified states can be subjected to optimization based on the desired constraint pose(s), the cost function (CF) and, optionally, the optimization constraints (OC) to define the control policy for haptic forces. Optionally, the result of this optimization can also be used to derive a tuning parameter (θc) to adjust the weights and biases of the neural network (NNopt), thereby providing real-time feedback enabling the neural network (NNopt) to optimize its identification policy to adjust to changing conditions.

The control policy is then utilized by the machine learning controller (MLC). The machine learning controller (MLC) can take as an input the prior forces, the prior constraint poses, and the desired constraint pose. Using the desired constraint pose as a target, the machine learning controller (MLC) can automatically adjust parameters based on feedback from prior values derived from the environment in attempt to optimize the refinement gains g(Kyd, Ku̅t, Ky̅t). The refinement gains (g) are then utilized to generate the refinement values for the haptic force, which can include a force refinement to be added to the haptic force component for each of the specified DOF intended to be constrained according to the active constraints.

### 2. Model- Free Predictive Control for Haptic Force Refinement

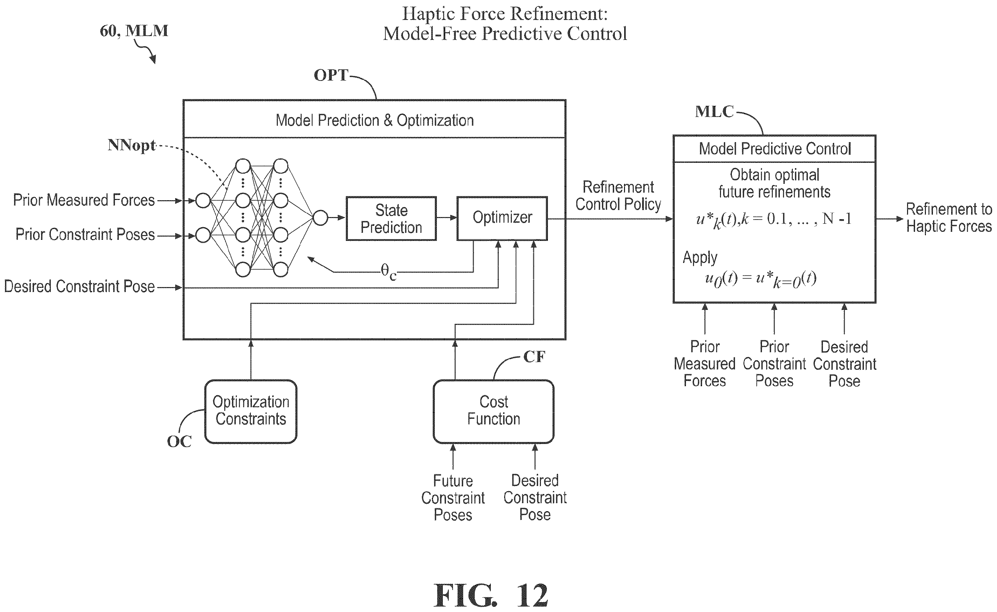

In another implementation, and with reference to FIG. 12, the machine learning model (MLM) is realized using a form of model-free predictive control (MFPC) or model-free reinforcement learning to generate the haptic force refinement. MFPC is configured to forecast control decisions by utilizing real-time (or prior) input-output data to make predictions and determine an optimal control policy. Similar to MFAC, the MFPC can be implemented using data-driven control, and therefore, can operate without requiring a detailed mathematical model of the state of the manipulator 14 or the environment in which the manipulator 14 interacts. Using MFPC, the control system 60 is able to adaptively and autonomously learn environmental interactions over time. MFPC similarly can operate using limited input data, e.g., the prior forces and constraint poses. Using MFPC, the machine learning model (MLM) can predict the states of the manipulator 14 from the measured input data and the control policy can be designed based on the linear state feedback control theory.

Referring to FIG. 12, one example of MFPC is illustrated wherein the optimizer (OPT) is realized as a model prediction and optimization block and the machine learning controller (MLC) is realized as a model predictive controller. In this example, the optimizer (OPT) is implemented with a neural network (NNopt), such as a deep-learning network. The neural network (NNopt) can receive, at the input layer, the prior forces and constraint poses, or variations thereof. The optimizer (OPT) also receives the desired constraint poses, although these values need not be inputted into the neural network. The neural network (NNopt) processes the prior measurements to generate an output that comprises a prediction of N future state(s) of the manipulator 14 (as shown at the "state prediction" block). The predicted state(s) can be used to "look ahead" to anticipate future uncertainties. In one example, the N future states can be N future constraint poses, which can be defined over a future horizon. The future horizon can be any suitable length, including up to a near-infinite horizon.

The optimizer (OPT) can automatically identify, generate, or update N future control policies. Namely, the N future states can be subjected to optimization based on the desired constraint pose(s), the cost function (CF) and, optionally, the optimization constraints (OC) to define N control policies or refinement control policies for haptic forces. The N control policies are sequence of policies that minimize the cost function (CF) over the given future horizon. The optimizer (OPT) solves the optimization problem at each control cycle, choosing the best control sequence over a "prediction horizon" to minimize the cost function (CF). Optionally, the result of this optimization can also be used to derive a tuning parameter (θc) to adjust the weights and biases of the neural network (NNopt), thereby providing real-time feedback enabling the neural network (NNopt) to optimize its predictive policy to adjust to changing conditions.

The N control policies are then utilized by the machine learning controller (MLC). The machine learning controller (MLC) can take as an input the prior forces, the prior constraint poses, and the desired constraint pose. The machine learning controller (MLC) can apply the described data to the N control policies to assess the haptic force refinement performance values of these policies. The machine learning controller (MLC) can utilize the same future horizon length used at the optimizer (OPT) or a future horizon of different length. The machine learning controller (MLC) derives haptic force refinement values based on first of the N future refinement control policies, disregarding the following ones, and this process repeats for the next time step at runtime. The machine learning controller (MLC) can base the control decision over a length of one, or longer.

For future time steps, the machine learning controller (MLC) can automatically adjust parameters based on experience from prior force/pose values derived from the environment in attempt to optimize policy evaluations. Based on the optimal future refinement, refinement values for the haptic force are generated, which can include a force refinement to be added to the haptic force component for each of the specified DOF intended to be constrained according to the active constraints.

### 3. Deep Reinforcement Learning for Haptic Force Refinement

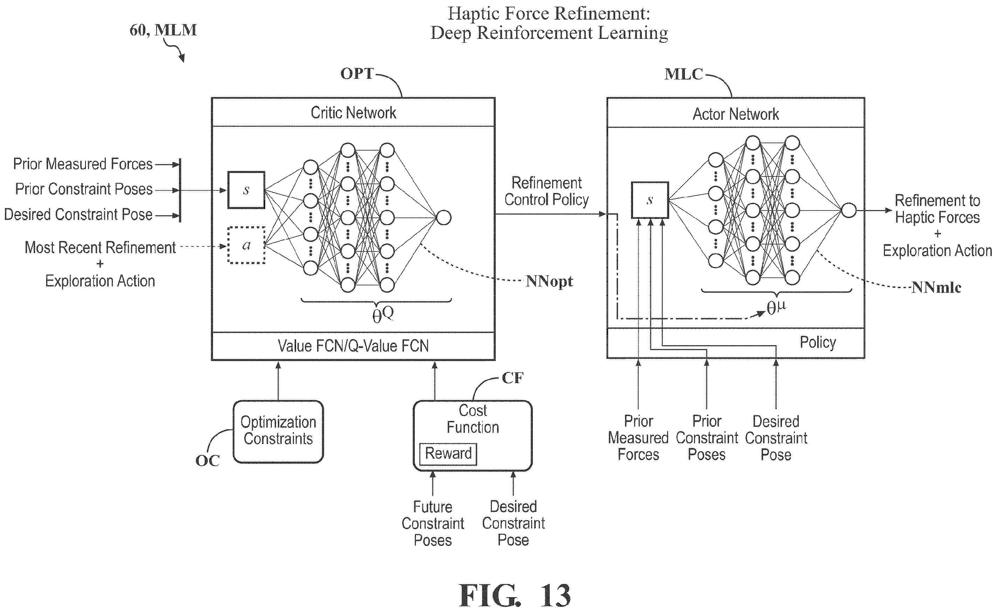

In another implementation, and with reference to FIG. 13, the machine learning model (MLM) is realized using a form of deep-reinforcement learning (DRL) to generate the haptic force refinement.

In this example, DRL is a machine learning technique that enables the model to make decisions that achieve optimal outcomes. DRL takes advantage of using deep learning (deep neural nets) in combination with reinforcement learning to speed up the learning process. Here, DRL can be implemented using data-driven control, and therefore, can operate without requiring a detailed mathematical model of the state of the manipulator 14 or the environment in which the manipulator 14 interacts. The DRL model described herein can provide a suitable solution for control in particular when dynamic interaction is not fully known. The DRL model can be implemented for continuous-time (CT) systems to achieve a near optimal performance.

The DRL model may be on-policy or off-policy. On-policy reinforcement learning updates the policy being used to take actions as the agent interacts with the environment. On the other hand, off-policy reinforcement learning is a model-free reinforcement learning algorithm that allows an agent to learn the value of the optimal policy independently of the agent's actions.

The DRL model described herein can be implemented in various forms, including but not limited to an Integral Reinforcement Learning (IRL) model, Deep Deterministic Policy Gradient (DDPG) model, Actor-Critic model, or Advantage Actor-Critic (A2C) models. IRL and DDPG models are both off-policy DRL techniques that use experience replay to learn optimal control solutions. IRL computes a "value function" that estimates the expected long-term reward an agent can achieve from a given state *(s).* On the other hand, DDPG is a Q-learning technique that is a specific algorithm used to learn the "Q-value function," which estimates the expected long-term reward for taking a particular action (*a*) in a specific state (s). Both IRL and DDPG algorithms can be implemented using the actor-critic networks where the actor applies a continuous control while the critic network incrementally corrects the actor's behavior until the optimal performance is achieved. Depending on the control model, the action (*a*) may not be utilized as the state (*s*) may be sufficient. Therefore, the action box (a) in FIG. 13 is illustrated as dotted to show that the action may be optional.

In FIG. 13, one example of a DRL model is illustrated wherein the optimizer (OPT) is realized as the critic network. The machine learning controller (MLC) is realized as the actor network. The critic network seeks to generate the refinement control policy and evaluate the quality of chosen actions, thereby providing feedback to the actor network.

In this example, the optimizer (OPT) is implemented with a neural network (NNopt), such as a deep-learning network. The neural network (NNopt) can receive, at the input layer, the prior forces, prior constraint poses, and optionally, the desired constraint poses. These values may be individually inputted to the neural network (NNopt) or may be combined prior to input to the neural network (NNopt). More specifically, these values may be inputted into input node(s) that are configured to receive the state *(s)* of the manipulator 14 and/or tool 20. The state in this example can be like any of the examples of the states of the manipulator 14 and/or surgical tool 20 described above. The states can be defined based on the residual errors between the desired constraint poses and the measured constraint poses, the derivative of the residual errors, the integral of the residual errors, and the like. The state (s) input is implemented in the DDPG and the IRL approach and can include presence of the described non-linear errors.

For the DDPG approach, the neural network additionally takes in the action (*a*)*,* to include a state-action input. In one implementation, the action (*a*) is the most recent (or last) haptic force refinement action combined with an exploration action that was previously decided by reinforcement learning from the actor network. The exploration action may be an epsilon random action the model (MLM) can deliberately choose with a higher probability, allowing discovery of the environment and potentially more optimal rewards. The randomness of the action can be defined by an epsilon value between 0 and 1 that controls the balance between exploration and exploitation, where a higher epsilon value denotes more random actions (exploration), and a lower epsilon value means more exploitation (choosing the currently best-known action). Increased exploration may be beneficial for the current solution due to the unknown system dynamics and non-linearities. The action (*a*) input can also include presence of the described non-linear errors.

The optimizer (OPT) utilizes the neural network (NNopt) to process the state (*s*) or state-action (*s, a*) input to automatically estimate the control policy. The control policy in this example can be achieved by utilizing the value function technique (to maximize the expected long-term reward the robotic system can achieve from a given state) or Q-value function (to maximize the expected long-term reward for the robotic system taking a particular action in a specific state). The neural network (NNopt) can make these function estimations by comparing a predicted value to an actual reward received. The cost function (CF) in this example can perform as a reward function. The cost function (CF) can be utilized to measure the model error based on this comparison and guide the learning of the neural network (NNopt) to adapt to changing conditions. The critic network perceives a reward from the environment if the previous action contributed to resolving the steady state error. The critic network seeks to maximize the return (its cumulative reward). The optimization constraints (OC) can additionally be utilized to limit the function estimations. Optionally, the result of this function estimation can also be used to derive a tuning parameter (θ^{Q}) to adjust the weights and biases of the neural network (NNopt), thereby providing real-time feedback enabling the neural network (NNopt) to optimize the value function to adjust to changing conditions.

The machine learning controller (MLC), implemented as the actor network, receives the control policy from the critic network. The actor network also can receive the current (or prior) state (s) values as an input, as described above. The actor network implements another neural network (NNmlc) that processes the state (s) value and determines the best action to take. The actor network does so based on the state *(s)* and refinement control policy. In turn, the actor network establishes a policy to map the states *(s)* to actions (*a*). As such, the actor network can also contribute to generating or modifying the refinement control policy. Using its policy, the actor network can generate the refinement values for the haptic force, which can include a force refinement to be added to the haptic force component for each of the specified DOF intended to be constrained according to the active constraints. The actor network may be configured to output refinement values as continuous action values, e.g., any haptic force refinement value within a continuous action space. In another implementation, the actor network outputs a probability distribution, e.g., a probability of selecting each available action. To implement the exploration action, the actor network can implement epsilon control parameters to inject noise into the outputted refinement. The control policy generated by the critic network is used to derive a tuning parameter (θµ) to adjust the weights and biases of the neural network (NNmlc), thereby providing real-time feedback enabling the neural network (NNmlc) to optimize its control policy to adjust to changing conditions. In turn, this update enables adaptive prediction defining how to combine the prior forces and constraint poses along with the desired constraint poses to generate the best action.

### C. Joint Torque Refinement

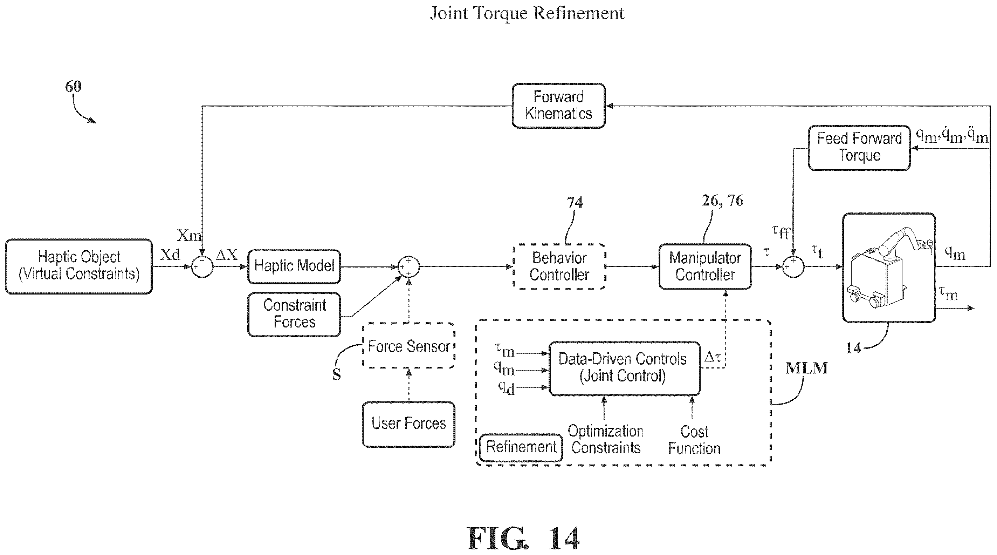

Referring to FIGS. 14-18, we now describe examples of the control system 60 implementing the various machine learning models (MLM) to improve performance of the robotic system by injecting a refinement (Δτ) to the commanded joint torques (τ) in attempt to more stiffly and accurately control the manipulator 14 and/or surgical tool 20.

Classical control approaches such as PIDs require a priori system knowledge in attempt to resolve steady state error for the robot manipulator within a specified working volume. In most cases, achieving the same level of accuracy throughout the entire robot workspace is challenging using one single PID controller while maintaining robot stability. Model-based controllers such as feed-forward torques can help to improve the performance of classical PID controllers so long as the model is a good representative of the robot dynamics. However, such models are not commonly feasible due to the presence of nonlinearity in the surgical robots especially for cable-driven manipulators. In the context of robotics-assisted arthroplasty, there is increasing interest to design a single platform to perform bone resection for multiple surgical applications. This desire requires the robot manipulator to be used in a different workspace for a particular operating room layout defined for that application. Therefore, having a single PID controller for bone resection for multiple surgical applications with a high accuracy and precision while maintaining the robot stability is almost impossible. Moreover, some applications such as TKA require multiple cuts with a substantial change in robot orientation. An optimal PID controller for one cut may not suit the others. Therefore, having one single PID controller tuned for the entire procedure is a considerable limitation. Such limitations may require designers to compromise the tuning of the controller to find a solution that works for all cuts, at the expense of extra controller error and a deterioration of the cutting accuracy for certain cuts.

In the joint space, the joints of the manipulator are commanded to move to joint positions by application of commanded torques on the respective actuators of the joints. However, non-linearities may cause actual joint positions to differ from the commanded joint positions. As a result, the joints fail to move as desired, potentially resulting in inaccuracies of the surgical tool and inability to address errors throughout the entire workspace of the manipulator. Similarly, traditional control approaches for surgical robotics fail to provide sufficient compensation for non-linear joint errors.

To provide a solution for some of the described shortcomings, the machine learning techniques described in this section minimize the error in the robot joint space by learning how the environment responds to certain control policies and what is the best future control policy for the given circumstance based on the behavior learned from the environment. The solutions provided herein achieve the required accuracy at the joint level for the entire robot workspace in the presence of robot nonlinear dynamics such as those resulting from variant inertia, friction, backlash, tool interactions, boundary variations, and other nonlinearities. The described techniques can learn the optimal control policy that suits a particular procedure, or all cuts with the expected accuracy.

With the proposed refinement solutions, the machine learning model (MLM) replaces the output of a classical PID controller with a more intelligent, predictive, and adaptive control scheme. The machine learning model (MLM) optimizes the cost function (CF) which is defined to achieve certain goals including a zero steady state error.

The prior sections have described various components, features, capabilities, terminology, and advantages related to the control system 60. For simplicity in description, the aforementioned will not be repeated in this section. However, it should be understood that any of the aforementioned are hereby fully incorporated in this section. Moreover, it is fully contemplated to combine the aspects of haptic force refinement with joint torque refinement or utilize these refinements separately.

Figure 14 provides a version of the control scheme of FIG. 6 modified by inclusion of the machine learning model (MLM) for joint torque refinement. To improve robotic system performance, the machine learning model (MLM) is injected into the joint torque computation pathway, which can include the manipulator controller 26 and/or motion controller 76. The machine learning model (MLM) is configured to output a refinement (Δτ) on the joint torques, which is combined with the commanded joint torques computed by the manipulator/motion controller(s) 26, 76. To provide this refinement, the machine learning model (MLM) takes measured inputs described in the preceding section. Namely, one input is the prior torques (τₘ) acting on the joint actuators, or otherwise derived from movement of, the surgical tool 20. Notably, the prior torques (τₘ) can include not only components of the commanded torques (τ) but also torque components derived from external disturbance(s) acting on the surgical tool 20 or manipulator 14, such as from tool-tissue interaction, collisions, human interaction, and the like. These external disturbances are usually the source of non-linear errors. Other inputs include prior (measured) joint position(s) (qₘ) and the desired joint position(s) (q_{d}). In addition to the measured inputs, the machine learning model (MLM) is also configured to utilize the optimization constraints (OC) and cost function (CF), which have been described above. Although the example in FIG. 14 illustrates supplementing the joint torque control scheme with the machine learning model (MLM), we reiterate that in an alternative implementation the machine learning model (MLM) can substitute partially or completely for the joint torque control scheme. In other words, the commanded torques (τ) can entirely be generated by the machine learning model (MLM).

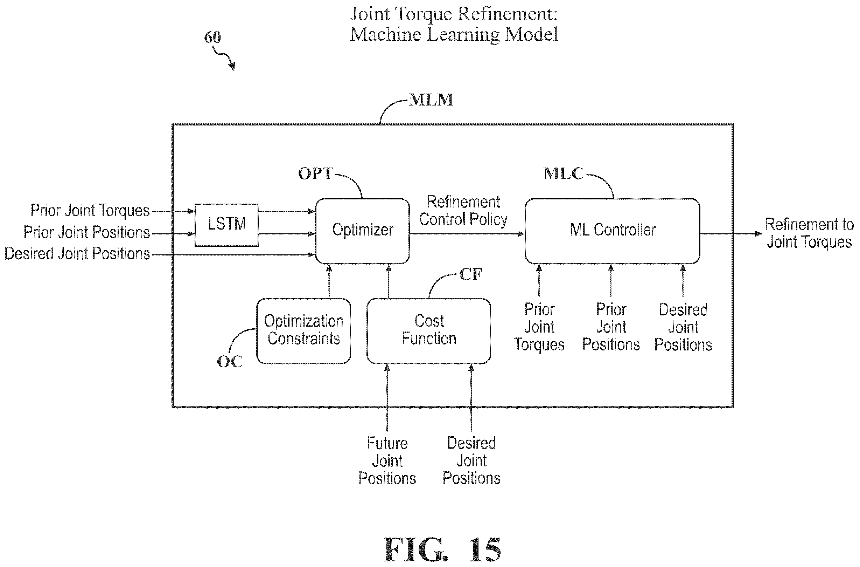

The diagram of FIG. 15 illustrates example architecture of the machine learning model (MLM) adapted for refining joint torque computation. The machine learning model (MLM) implements the optimizer (OPT) to receive the input values, i.e., prior joint position value(s) (qₘ), the prior joint torque value(s) (Tₘ), and the desired joint position value(s) (q_{d}). In one example, the prior joint position and prior joint torque values are directly inputted into the optimizer (OPT). In another implementation, prior joint position and prior joint torque values are optionally pre-processed by the LSTM prior to being inputted into the optimizer (OPT).

The future joint position value(s) (q_{f}) and desired joint position value(s) (q_{q}) are inputted into the cost function (CF). The optimizer (OPT) also receives the model error outputted by the cost function (CF). The optimizer (OPT) can also be subjected to optimization constraints (OC). The optimizer (OPT) processes the prior joint position and torque value(s) and desired joint position value(s) while optionally being subject to optimization constraints (OC). Based on the incoming data, the optimizer (OPT) defines the refinement control policy that optimizes the cost function (CF) and that defines the rules used to refine the joint torques. One goal of the optimizer (OPT) is to determine the most optimal refinement control policy to achieve the desired joint positions (q_{d}) of the manipulator 14. The machine learning controller (MLC) can take as an input the prior joint torque value(s) and the prior joint position value(s). In some cases, the machine learning controller (MLC) optionally can take in the desired joint position value(s). These prior and desired value(s) can be applied to the refinement control policy generated by the optimizer (OPT) in order to determine the refinement on joint torques. The refinement of the joint torques is utilized to control the manipulator 14 to move to refined joint positions in attempt to achieve the desired joint positions. The commanded joint torques can include a torque component for each of active joint(s) (J) of the manipulator 14. The refinement can include a torque refinement to be added to the computed torque component for each of the active joint(s).

Figure 19 provides example equations and expressions (1')(2')(3') and (4)-(6) that the control system 60 can utilize in joint torque refinement calculations for any of the implementations described herein. Equation (1') expresses an output vector u(t) of the prior joint torque value(s) (τₘ) as a function of time. Equation (2') expresses an output vector y(t) of the prior joint position value(s) (qₘ) as a function of time. Equation (3') expresses a vector yd(t) of the desired joint position value(s) (q_{d}) as a function of time. Equation (4) represents N samples of the output vector of the prior joint torque value(s) (u(t)), where u^{T} is the transpose of the vector u(t). Equation (5) represents N samples of the output vector of the prior joint position value(s) (y(t)), where y^{T} is the transpose of the vector y(t). These equations and expressions are provided as examples and are not intended to limit the scope of the described solutions.

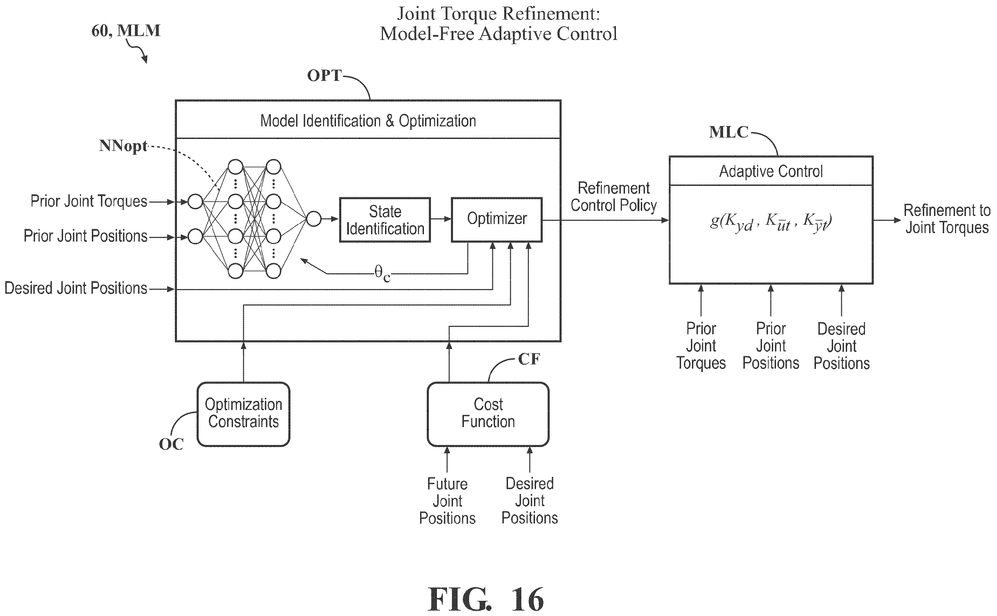

As will be described in the following examples of FIGS. 16-18, the machine learning model (MLM) adapted for joint torque refinement will be described using various architectures, such as, but not limited to: model-free adaptive control (MFAC), model-free predictive control (MFPC), and deep refinement learning (DRL), and equivalents or combinations thereof. Any of these machine learning frameworks may be realized as a data-driven system. These machine-learning based frameworks aim at learning what is the best control policy for the given circumstances based on the behavior learned from the environment and understanding how the environment responds to certain haptic control policies. The diagrams of FIG. 14 and 15 can be understood to as a functional generalization that can embody any of the described examples. The proposed solutions are not limited to any particular example described herein and may be captured more as set forth generally.

### 1. Model- Free Adaptive Control for Joint Torque Refinement

In one implementation, and with reference to FIG. 16, the machine learning model (MLM) is realized using a form of model-free adaptive control (MFAC) to generate the joint torque refinement.

Here, the optimizer (OPT) is realized as a model identification and optimization block and the machine learning controller (MLC) is realized as an adaptive controller. The optimizer (OPT) is implemented with a neural network (NNopt), such as a deep-learning network. The neural network (NNopt) can receive, at the input layer, the prior joint torques and prior joint positions, or variations thereof. The optimizer (OPT) also receives the desired joint positions, although these values are not required to be inputted into the neural network.

The neural network (NNopt) processes the prior measurements to generate an output that comprises an identification of one or more state(s) of the manipulator 14 (as shown at the "state identification" block). Notably, the identified state of the manipulator 14 may be identified in the presence of the described non-linearities. The state of the manipulator 14 in this sense can be a state that is not otherwise easily identified or predicted based on the prior values and can be used as a surrogate to a predetermined system model of the manipulator 14. Although prior values are utilized, the identified state of the manipulator 14 are generated to account for changing conditions arising from uncertainties or non-linearities in the task space. In one example, the state of the manipulator identifies an actual or next joint position for any number of joint(s).

The optimizer (OPT) can automatically identify, generate, or update the control policy for the joint torques. Namely, the identified states can be subjected to optimization based on the desired joint position(s), the cost function (CF) and, optionally, the optimization constraints (OC) to define the control policy for joint torques. Optionally, the result of this optimization can also be used to derive a tuning parameter (θc) to adjust the weights and biases of the neural network (NNopt), thereby providing real-time feedback enabling the neural network (NNopt) to optimize its identification policy to adjust to changing conditions.

The control policy is then utilized by the machine learning controller (MLC). The machine learning controller (MLC) can take as an input the prior joint torque(s), the prior joint position(s), and the desired joint position(s). Using the desired joint position(s) as a target, the machine learning controller (MLC) can automatically adjust parameters based on feedback from prior values derived from the environment in attempt to optimize the refinement gains g(Kyd, Ku̅t, Ky̅t). The refinement gains (g) are then utilized to generate the refinement values for the joint torque, which can include the torque refinement to be added to the computed torque value for each active joint of the manipulator 14.

### 2. Model- Free Predictive Control for Joint Torque Refinement

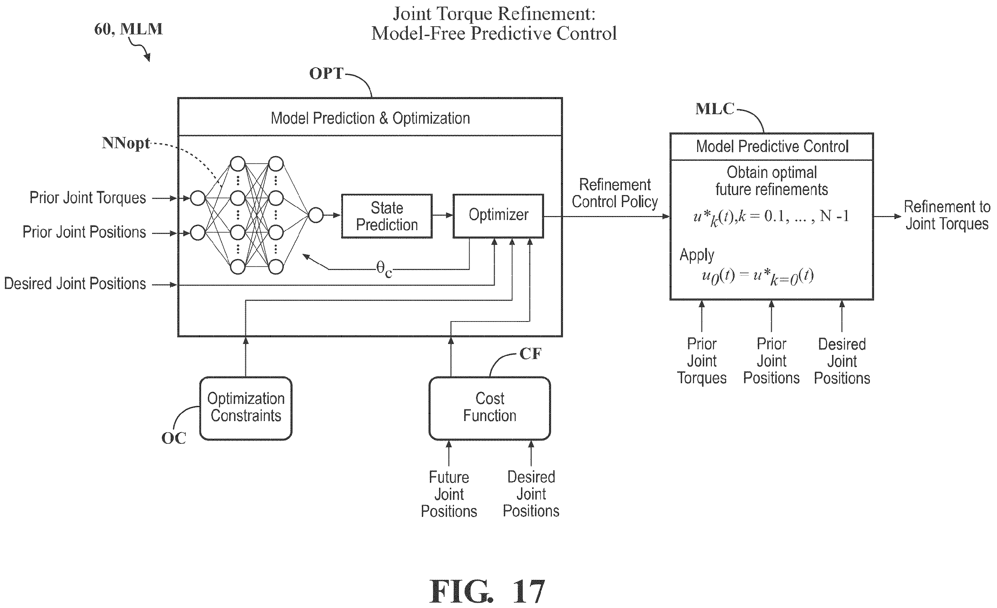

In another implementation, and with reference to FIG. 17, the machine learning model (MLM) is realized using a form of model-free predictive control (MFPC) or model-free reinforcement learning to generate the joint torque refinement. Here, the optimizer (OPT) is realized as a model prediction and optimization block and the machine learning controller (MLC) is realized as a model predictive controller. The optimizer (OPT) is implemented with a neural network (NNopt), such as a deep-learning network. The neural network (NNopt) can receive, at the input layer, the prior joint torques and prior joint positions, or variations thereof. The optimizer (OPT) also receives the desired joint positions, although these values are need not be inputted into the neural network.

The neural network (NNopt) processes the prior measurements to generate an output that comprises a prediction of N future state(s) of the manipulator 14 (as shown at the "state prediction" block). The predicted state(s) can be used to "look ahead" to anticipate future uncertainties. In one example, the N future states can be N future joint positions, which can be defined over a future horizon. The future horizon can be any suitable length, including up to a near-infinite horizon.

The optimizer (OPT) can automatically identify, generate, or update N future control policies. Namely, the N future states can be subjected to optimization based on the desired joint position(s), the cost function (CF) and, optionally, the optimization constraints (OC) to define N control policies or refinement control policies for joint torques. The N control policies are sequence of policies that minimize the cost function (CF) over the given future horizon. The optimizer (OPT) solves the optimization problem at each control cycle, choosing the best control sequence over a "prediction horizon" to minimize the cost function (CF). Optionally, the result of this optimization can also be used to derive a tuning parameter (θc) to adjust the weights and biases of the neural network (NNopt), thereby providing real-time feedback enabling the neural network (NNopt) to optimize its predictive policy to adjust to changing conditions.

The N control policies are then utilized by the machine learning controller (MLC). The machine learning controller (MLC) can take as an input the prior joint torques, the prior joint positions, and the desired joint position. The machine learning controller (MLC) can apply the described data to the N control policies to assess the joint torque refinement performance values of these policies. The machine learning controller (MLC) can utilize the same future horizon length used at the optimizer (OPT) or a future horizon of different length. The machine learning controller (MLC) derives joint torque refinement values based on first of the N future refinement control policies, disregarding the following ones, and this process repeats for the next time step at runtime. The machine learning controller (MLC) can base the control decision over a length of one, or longer.

For future time steps, the machine learning controller (MLC) can automatically adjust parameters based on experience from prior torque/position values derived from the environment in attempt to optimize policy evaluations. Based on the optimal future refinement, refinement values for the joint torque are generated.

### 3. Deep Reinforcement Learning for Joint Torque Refinement

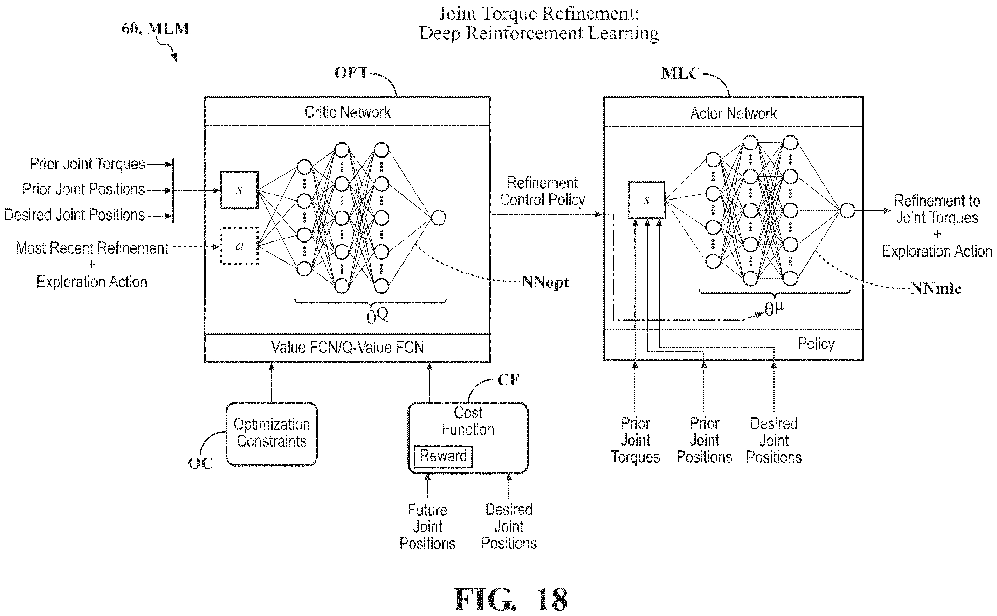

In another implementation, and with reference to FIG. 18, the machine learning model (MLM) is realized using a form of deep-reinforcement learning (DRL) to generate the joint torque refinement. Here, the optimizer (OPT) is realized as a critic network. The machine learning controller (MLC) is realized as an actor network. The critic network seeks to generate the refinement control policy and evaluate the quality of chosen actions, thereby providing feedback to the actor network.

The optimizer (OPT) is implemented with a neural network (NNopt), such as a deep-learning network. The neural network (NNopt) can receive, at the input layer, the prior joint torques, prior joint positions, and optionally, the desired joint positions. These values may be individually inputted to the neural network (NNopt) or may be combined prior to input to the to the neural network (NNopt). More specifically, these values may be inputted into input node(s) that are configured to receive the state *(s)* of the manipulator 14 and/or tool 20. The state in this example can be like any of the examples of the states of the manipulator 14 and/or surgical tool 20 described above. The states can be defined based on the residual errors between the desired joint positions and the measured joint positions, the derivative of the residual errors, the integral of the residual errors, and the like. The state (s) input is implemented in the DDPG and the IRL approach and can include presence of the described non-linear errors.

For the DDPG approach, the neural network additionally takes in the action (*a*)*,* to include a state-action input. In one implementation, the action (*a*) is the most recent (or last) joint torque refinement action combined with an exploration action that was previously decided by reinforcement learning from the actor network. As described in the prior section, the exploration action may be an epsilon random action. The action (*a*) input can also include presence of the described non-linear errors.

The optimizer (OPT) utilizes the neural network (NNopt) to process the state (*s*) or state-action (*s, a*) input to automatically estimate the control policy. The control policy in this example can be achieved by utilizing the value function technique (to maximize the expected long-term reward the robotic system can achieve from a given state) or Q-value function (to maximize the expected long-term reward for the robotic system taking a particular action in a specific state). The neural network (NNopt) can make these function estimations by comparing a predicted value to an actual reward received. The cost function (CF) in this example can perform as a reward function. The cost function (CF) can be utilized to measure the model error based on this comparison and guide the learning of the neural network (NNopt) to adapt to changing conditions. The critic network perceives a reward from the environment if the previous action contributed to resolving the steady state error. The critic network seeks to maximize the return (its cumulative reward). The optimization constraints (OC) can additionally be utilized to limit the function estimations. Optionally, the result of this function estimation can also be used to derive a tuning parameter (θ^{Q}) to adjust the weights and biases of the neural network (NNopt), thereby providing real-time feedback enabling the neural network (NNopt) to optimize the value function to adjust to changing conditions.

The machine learning controller (MLC), implemented as the actor network, receives the control policy from the critic network. The actor network also can receive the current (or prior) state (s) values as an input, as described above. The actor network implements another neural network (NNmlc) that processes the state (s) value and determines the best action to take. The actor network does so based on the state *(s)* and refinement control policy. In turn, the actor network establishes a policy to map the states *(s)* to actions (*a*). As such, the actor network can also contribute to generating or modifying the refinement control policy. Using its policy, the actor network can generate the refinement values for the joint torque. The actor network may be configured to output refinement values as continuous action values, e.g., any joint torque refinement value within a continuous action space. In another implementation, the actor network outputs a probability distribution, e.g., a probability of selecting each available action. To implement the exploration action, the actor network can implement epsilon control parameters to inject noise into the outputted refinement. The control policy generated by the critic network is used to derive a tuning parameter (θµ) to adjust the weights and biases of the neural network (NNmlc), thereby providing real-time feedback enabling the neural network (NNmlc) to optimize its control policy to adjust to changing conditions. In turn, this update enables adaptive prediction defining how to combine the prior forces and constraint poses along with the desired constraint poses to generate the best action.

Several embodiments have been described in the foregoing description. However, the embodiments discussed herein are not intended to be exhaustive or limit the invention to any particular form. The terminology, which has been utilized, is intended to be in the nature of words of description rather than of limitation. Many modifications and variations are possible in light of the above teachings and the invention may be practiced otherwise than as specifically described.

The present disclosure also comprises the following clauses, with specific features laid out in dependent clauses, that may specifically be implemented as described in greater detail with reference to the configurations and drawings above.

### CLAUSES

C1. A surgical system comprising: a robotic manipulator comprising a plurality of links and joints; a control system coupled to the robotic manipulator and being configured to: compute joint torques for the joints of the robotic manipulator; control the joints of the robotic manipulator in accordance with the computed joint torques to move the joints to joint positions; acquire prior measured joint torques; acquire prior measured joint positions; obtain desired joint positions for the joints; and input the prior measured joint torques, the prior measured joint positions, and the desired joint positions into a machine learning model that is configured to optimize a cost function and generate an output comprising a refinement to the computed joint torques in attempt to achieve the desired joint positions.
C2. The surgical system of clause C1, wherein the machine learning model is a data-driven control model that is configured to define a refinement control policy based on the prior measured joint torques and the prior measured joint positions.
C3. The surgical system of any preceding clause, wherein the machine learning model comprises one of: a model-free adaptive control model; and a model-free predictive control model.
C4. The surgical system of any preceding clause, wherein the machine learning model comprises a deep reinforcement learning model.
C5. The surgical system of any preceding clause, wherein: the machine learning model comprises a neural network; and the control system is further configured to input the prior measured joint torques and the prior measured joint positions into the neural network.
C6. The surgical system of clause C5, wherein the machine learning model is configured to define a refinement control policy.
C7. The surgical system of clause C6, wherein the machine learning model is configured to generate the refinement by being configured to apply the prior measured joint torques, the prior measured joint positions, and the desired joint positions to the refinement control policy.
C8. The surgical system of clause C7, wherein: the neural network is configured to generate an output that comprises an identification of a state of the robotic manipulator; the control system is configured to further input the desired joint positions into the machine learning model; and the machine learning model is configured to implement an optimizer that is configured to input the identified state and the desired joint positions into the cost function and optimize the cost function to define the refinement control policy.
C9. The surgical system of clause C7, wherein: the neural network is configured to generate an output that comprises predicted states of the robotic manipulator over a defined future horizon; the control system is configured to further input the desired joint positions into the machine learning model; the machine learning model is configured to implement an optimizer that is configured to: input the predicted states and the desired joint positions into the cost function and optimize the cost function to define N future refinement control policies; and select the refinement control policy based on a first of the N future refinement control policies.
C10. The surgical system of clause C7, wherein: the control system is further configured to: obtain a most-recent refinement to the computed joint torques; and input the most-recent refinement into the neural network; and the neural network is configured to process the prior measured joint torques, the prior measured joint positions, the desired joint positions, and the most-recent refinement to define the refinement control policy.
C11. The surgical system of any preceding clause, wherein the control system is further configured to: predict future joint positions and input the future joint positions into the machine learning model; and utilize the machine learning model to optimize the cost function based on the desired joint positions and the future joint positions to determine one or more future refinements to the computed joint torques.
C12. The surgical system of clause C11, wherein the control system utilizes the machine learning model to optimize the cost function by being configured to minimize one or more of: future residual errors, oscillation in joint positions, and energy injected into the robotic manipulator.
C13. The surgical system of any preceding clause, wherein: the computed joint torques comprise a torque value for each active joint of the robotic manipulator; and the refinement comprises a torque refinement to be added to the torque value for each active joint of the robotic manipulator.
C14. The surgical system of any preceding clause, wherein the control system is configured to: apply a range defining a minimum value and a maximum value for the refinement; and apply the refinement to the computed joint torques only in response to the refinement falling within the range.
C15. The surgical system of any preceding clause, wherein the control system comprises a long short-term memory (LSTM) coupled to an input of the machine learning model, wherein the LSTM comprises a recurrent neural network (RNN) and is configured to: receive values of the prior measured joint torques and values of the prior measured joint positions; and modify weights of the RNN to learn long-term dependencies among the values and to selectively retain or discard the values.
C16. The surgical system of any preceding clause, wherein the prior measured joint torques include torques derived from: the computed joint torques and external disturbance(s) acting on the surgical tool or the robotic manipulator.
C17. The surgical system of any preceding clause, wherein the control system is configured to: operate the robotic manipulator in a manual mode wherein the robotic manipulator is configured to move the surgical tool responsive to external forces/torques applied to the surgical tool by a user; and in the manual mode, generate the refinement to the computed joint torques in attempt to achieve the desired joint positions.
C18. The surgical system of any preceding clause, wherein the control system is configured to: operate the robotic manipulator in an automated mode wherein the robotic manipulator is configured to automatically move the surgical tool along a predetermined tool path; and in the automated mode, generate the refinement to the computed joint torques in attempt to achieve the desired joint positions.
C19. A method of operating the surgical system of any preceding clause.
C20. A computer program product comprising instructions, which when executed by one or more processors, are configured to operate the surgical system of any preceding clause.

## Claims

1. A surgical system (10) comprising:
a robotic manipulator (14) comprising a plurality of links (18) and joints (J);
a surgical tool (20) coupled to the robotic manipulator (14);
a control system (60) coupled to the robotic manipulator (14) and being configured to:
compute haptic forces (Factive) that are intended to constrain specified degrees of freedom (DOF) of the surgical tool (20);
based on the haptic forces (Factive), generate commanded joint torques (τ) to control the robotic manipulator (14) to move to constraint poses (Xm) in attempt to actively constrain the specified DOF of the surgical tool (20);
measure actual joint torques (τm) generated responsive to movement of the robotic manipulator (14) to the constraint poses (Xm);
convert the actual joint torques (τm) into measured forces (Fm_active) for the specified DOF;
acquire prior measured forces (Fm_active);
acquire prior constraint poses (Xm);
obtain a desired constraint pose (Xd) of the surgical tool (20) according to the specified DOF; and
input the prior measured forces (Fm_active), the prior constraint poses (Xm), and the desired constraint pose (Xd) into a machine learning model (MLM) that is configured to optimize a cost function (CF) and generate an output comprising a refinement (ΔFactive) to the haptic forces (Factive) in attempt to achieve the desired constraint pose (Xd) of the surgical tool (20) according to the specified DOF.

2. The surgical system (10) of any preceding claim, wherein the machine learning model (MLM) is a data-driven control model that is configured to define a refinement control policy based on the prior measured forces (Fm_active) and the prior constraint poses (Xm).

3. The surgical system (10) of any preceding claim, wherein:
the machine learning model (MLM) comprises a neural network (NNopt); and
the control system (60) is further configured to input the prior measured forces (Fm_active) and the prior constraint poses (Xm) into the neural network (NNopt).

4. The surgical system (10) of claim 3, wherein the machine learning model (MLM) is configured to:
define a refinement control policy; and
generate the refinement (ΔFactive) by applying the prior measured forces (Fm_active), the prior constraint poses (Xm), and the desired constraint pose (Xd) to the refinement control policy.

5. The surgical system (10) of claim 4, wherein:
the neural network (NNopt) is configured to generate an output that comprises an identification of a state of the robotic manipulator (14);
the control system (60) is configured to further input the desired constraint pose (Xd) into the machine learning model (MLM); and
the machine learning model (MLM) is configured to implement an optimizer (OPT) that is configured to input the identified state and the desired constraint pose (Xd) into the cost function (CF) and optimize the cost function (CF) to define the refinement control policy.

6. The surgical system (10) of claim 4, wherein:
the neural network (NNopt) is configured to generate an output that comprises predicted states of the robotic manipulator (14) over a defined future horizon;
the control system (60) is configured to further input the desired constraint pose (Xd) into the machine learning model (MLM); and
the machine learning model (MLM) is configured to implement an optimizer (OPT) that is configured to:
input the predicted states and the desired constraint pose (Xd) into the cost function (CF) and optimize the cost function (CF) to define N future refinement control policies; and
select the refinement control policy based on a first of the N future refinement control policies.

7. The surgical system (10) of claim 4, wherein:
the control system (60) is further configured to:
obtain a most-recent refinement (ΔFactive) to the haptic forces (Factive); and
input the most-recent refinement (ΔFactive) into the neural network (NNopt); and
the neural network (NNopt) is configured to process the prior measured forces (Fm_active), the prior constraint poses (Xm), the desired constraint pose (Xd), and the most-recent refinement (ΔFactive) to define the refinement control policy.

8. The surgical system (10) of any preceding claim, wherein the control system (60) is further configured to:
predict future constraint poses (Xm) and input the future constraint poses (Xm) into the machine learning model (MLM); and
utilize the machine learning model (MLM) to optimize the cost function (CF) based on the desired constraint pose (Xd) and the future constraint poses (Xm) to determine one or more future refinements (ΔFactive) to the haptic forces (Factive) in attempt to achieve the desired constraint pose (Xd) of the surgical tool (20) according to the specified DOF.

9. The surgical system (10) of any preceding claim, wherein:
the haptic forces (Factive) comprise a haptic force component for each of the specified DOF; and
the refinement (ΔFactive) comprises a force refinement to be added to the haptic force component for each of the specified DOF.

10. The surgical system (10) of any preceding claim, wherein the control system (60) is configured to:
apply a range defining a minimum value and a maximum value for the refinement (ΔFactive); and
apply the refinement (ΔFactive) to the haptic forces (Factive) only in response to the refinement (ΔFactive) falling within the range.

11. The surgical system (10) of any preceding claim, wherein the control system (60) comprises a long short-term memory (LSTM) coupled to an input of the machine learning model (MLM), wherein the LSTM comprises a recurrent neural network (RNN) and is configured to:
receive values of the prior measured forces (Fm_active) and values of the prior constraint poses (Xm); and
modify weights of the RNN to learn long-term dependencies among the values and to selectively retain or discard the values.

12. The surgical system (10) of any preceding claim, wherein:
the control system (60) implements a haptic control model (HMa) that is configured to compute the haptic forces (Factive) that are intended to constrain the specified DOF of the surgical tool (20) in a task space of the robotic manipulator (14); and
the haptic control model (HMa) is configured to compute the haptic forces (Factive) that are intended to constrain the specified DOF of the surgical tool (20) relative to a haptic object.

13. The surgical system (10) of claim 12, wherein:
the haptic object is a haptic line, the haptic control model (HMa) is configured to compute the haptic forces (Factive) that are intended to constrain at least four specified DOF of the surgical tool (20) relative to the haptic line, and the at least four specified DOF comprise at least two rotational DOF and two translational DOF; or
the haptic object is a haptic plane, the haptic control model (HMa) is configured to compute the haptic forces (Factive) that are intended to constrain at least three specified DOF of the surgical tool (20) relative to the haptic plane, and the at least three specified DOF comprise two rotational DOF and at least one translational DOF; or
the haptic object is a haptic volume, the haptic control model (HMa) is configured to compute the haptic forces (Factive) that are intended to constrain up to three specified DOF of the surgical tool (20) relative to the haptic volume, and the up to three specified DOF comprise up to three rotational DOF.

14. The surgical system (10) of any preceding claim, wherein the control system (60) is configured to:
operate the robotic manipulator (14) in a manual mode wherein the robotic manipulator (14) is configured to move the surgical tool (20) responsive to external forces/torques applied to the surgical tool (20) by a user and attempt to actively constrain the specified DOF of the surgical tool (20) during operation in the manual mode; or
operate the robotic manipulator (14) in an automated mode wherein the robotic manipulator (14) is configured to automatically move the surgical tool (20) along a predetermined tool path and attempt to actively constrain the specified DOF of the surgical tool (20) during operation in the automated mode.

15. A method of operating the surgical system (10) of any preceding claim.
